(19) Europäisches Patentamt
European Patent Office
Office européen des brevets

(11) Numéro de publication : **0 082 049**
**B1**

(12) **FASCICULE DE BREVET EUROPÉEN**

(45) Date de publication du fascicule du brevet : **29.07.87**

(21) Numéro de dépôt : **82402211.5**

(22) Date de dépôt : **03.12.82**

(51) Int. Cl.⁴ : **C 07 D307/93**, C 07 D307/77, C 07 B 57/00, C 07 D405/12, C 07 C121/75, C 07 D213/64, C 07 C 49/743, C 07 C 31/125// C07C62/02

(54) **Nouveaux éthers dont les restes organiques comportent des atomes chiraux, leur préparation, leur application au dédoublement d'alcools ou de certains composés hémiacétaliques et les nouveaux produits dédoublés obtenus.**

(30) Priorité : **09.12.81 FR 8123003**

(43) Date de publication de la demande : **22.06.83 Bulletin 83/25**

(45) Mention de la délivrance du brevet : **29.07.87 Bulletin 87/31**

(84) Etats contractants désignés : **AT BE CH DE FR GB IT LI NL SE**

(56) Documents cités :
**EP-A- 0 041 021**
**FR-A- 2 382 426**
**FR-A- 2 383 927**
**FR-A- 2 423 488**

(73) Titulaire : **ROUSSEL-UCLAF**
**35, boulevard des Invalides**
**F-75007 Paris (FR)**

(72) Inventeur : **Tessier, Jean**
**30, rue Jean Moulin**
**F-94300 Vincennes (FR)**
Inventeur : **Demoute, Jean-Pierre**
**249bis, rue de Rosny**
**F-93100 Montreuil-sous-Bois (FR)**

(74) Mandataire : **Tonnellier, Marie-José et al**
**Département des Brevets ROUSSEL UCLAF B.P. no 9**
**F-93230 Romainville (FR)**

**EP 0 082 049 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux éthers dont les restes organiques comportent des atomes chiraux, leur procédé de préparation, leur application au dédoublement d'alcools ou de certains composés de structure hémiacétalique et les nouveaux produits dédoublés obtenus.

L'invention a plus précisément pour objet sous toutes les formes isomères possibles, les composés de formule générale I :

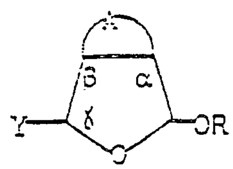

(I)

formule dans laquelle le symbole A représente une chaîne hydrocarbonée comportant de 1 à 16 chaînons, cette chaîne pouvant comporter un ou plusieurs hétéroatomes, une ou plusieurs insaturations, l'ensemble des chaînons constitutifs de la chaîne pouvant représenter un système mono ou polycyclique, y compris un système du type spiro ou endo, l'ensemble constitué par la chaîne A et les atomes de carbone auxquels elle est liée, pouvant comporter un ou plusieurs atomes chiraux ou bien la copule hémiacétalique pouvant présenter une chiralité due à la configuration spatiale dissymétrique de l'ensemble de la molécule, R représente soit un reste d'alcool primaire, secondaire ou tertiaire, comportant au moins un carbone asymétrique, soit un reste alcoolique substitué dont la chiralité est due à la configuration spatiale dissymétrique de l'ensemble de la molécule, Y représente soit un atome d'hydrogène, soit un groupement $-CY'_3$ dans lequel Y' est un atome de chlore ou de brome, soit un groupement alcoyle, linéaire ou ramifié, comportant de 1 à 18 atomes de carbone éventuellement substitué, la liaison $\beta\gamma$ de la formule I ainsi que le substituant Y pouvant dans ce dernier cas faire partie du substituant A, ainsi que les mélanges de ces isomères.

Dans les composés de formule (I) selon l'invention, Y représente notamment un méthyle, un éthyle, un propyle, linéaire ou ramifié, un butyle, linéaire ou ramifié, un pentyle, linéaire ou ramifié, un hexyle, linéaire ou ramifié, un heptyle, linéaire ou ramifié, un octyle, linéaire ou ramifié, un nonyle, linéaire ou ramifié, un décyle, linéaire ou ramifié.

Lorsque Y représente un radical alcoyle substitué, il s'agit de préférence d'un radical alcoyle substitué par un atome d'halogène, comme, par exemple, un atome de brome ou de chlore.

Dans les composés de formule (I) selon l'invention, les deux radicaux différents qui substituent le ou les centres asymétriques de la chaîne A ou les atomes de carbone auxquels elle est liée, sont notamment un méthyle, un éthyle, un propyle, linéaire ou ramifié, un butyle, linéaire ou ramifié, un pentyle, linéaire ou ramifié, un hexyle, linéaire ou ramifié, un heptyle, linéaire ou ramifié, un octyle, linéaire ou ramifié, un nonyle, linéaire ou ramifié, un décyle, linéaire ou ramifié ou forment ensemble avec l'atome de carbone auquel ils sont liés, un cyclopropyle, un cyclobutyle, un cyclopentyle, un cyclohexyle.

Parmi les composés préférés de l'invention, on peut citer les composés de formule (I) dans lesquels les atomes ou radicaux différents qui substituent le ou les atomes de carbone portés par la chaîne A ou situés en $\alpha$ ou $\beta$, sont choisis indifféremment dans l'un ou l'autre des groupes suivants :

a) le groupe constitué par l'hydrogène, les atomes d'halogènes, le groupement nitro, les radicaux alcoyles comportant de 1 à 18 atomes de carbone, les radicaux cyclo alcoyles comportant de 3 à 6 atomes de carbone, le radical phényle, les radicaux phényles substitués soit par un ou plusieurs atomes d'halogènes, soit par un ou plusieurs radicaux alcoyles comportant de 1 à 6 atomes de carbone ;

b) le groupe constitué par les radicaux :

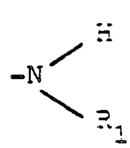

dans lesquels $R_1$ représente un atome d'hydrogène, un radical alcoyle comportant de 1 à 6 atomes de carbone, le radical :

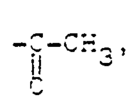

le radical :

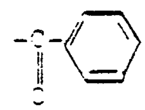

ou le radical :

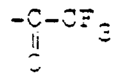

c) le groupe constitué par les radicaux :

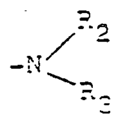

dans lesquels $R_2$ et $R_3$, identiques ou différents, représentent un radical alcoyle comportant de 1 à 6 atomes de carbone ou bien dans lesquels $R_2$ et $R_3$ représentent ensemble avec l'atome d'azote auquel ils sont liés, un hétérocycle comportant 6 atomes.

Dans les composés de formule I préférés selon l'invention, les radicaux alcoyles qui substituent le radical phényle sont de préférence choisis parmi les radicaux méthyle, éthyle, propyle, linéaire ou ramifié, butyle, linéaire ou ramifié, pentyle, linéaire ou ramifié et hexyle, linéaire ou ramifié.

Dans les composés de formule I préférés, $R_1$, $R_2$ et $R_3$ représentent notamment un méthyle, un éthyle, un propyle, linéaire ou ramifié, un butyle, linéaire ou ramifié, un pentyle, linéaire ou ramifié, un hexyle, linéaire ou ramifié, ou $R_2$ et $R_3$ représentent notamment avec l'atome d'azote auquel ils sont liés, un groupement pyridinyle, pyridazinyle, pyrimidinyle, pyrazolinyle, pipérazinyle, triazinyle, ou oxazinyle.

Comme composés de formule I particulièrement intéressants, on peut signaler ceux qui sont caractérisés en ce que la chaîne A a pour structure soit :

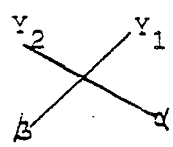

$Y_1$ et $Y_2$, identiques ou différents, représentant un atome d'hydrogène, de fluor, de chlore ou de brome ou un radical alcoyle comportant de 1 à 6 atomes de carbone, $Y_1$ et $Y_2$ pouvant représenter notamment un radical méthyle ou $Y_1$ et $Y_2$ formant ensemble avec l'atome de carbone auquel ils sont liés, un homocycle carboné comportant de 3 à 7 atomes de carbone,
soit :

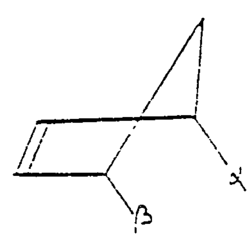

soit :

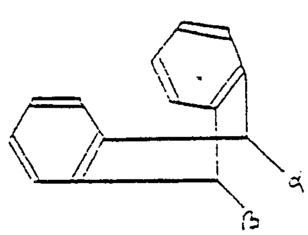

soit :

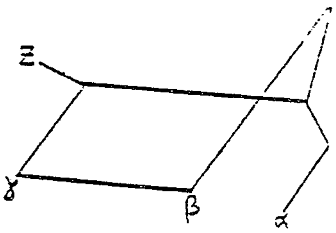

formule dans laquelle Z représente un atome d'hydrogène, un atome de chlore, un atome de brome ou d'iode.

Dans les composés de formule I, R peut représenter le reste d'un alcool primaire, secondaire ou tertiaire de nature aliphatique, cycloaliphatique ou aromatique, mono ou polycyclique.

Comme composés de formule I particulièrement intéressants on peut citer ceux dans lesquels R représente un reste cyano méthyle substitué choisi dans le groupe constitué par les radicaux :

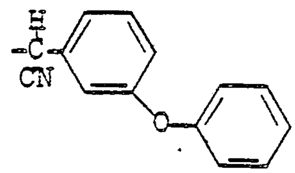 , 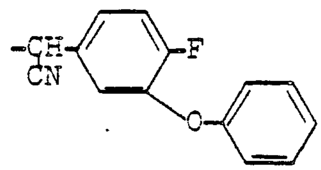 et 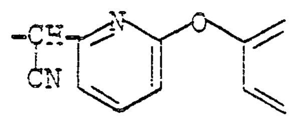

On peut citer également les composés de formule I correspondants, dans lesquels le groupement cyano est remplacé par un radical alkyle, alkényle, ou alkynyle.

Comme autres composés particulièrement utiles, on peut citer les composés de formule I, dans lesquels R représente le radical :

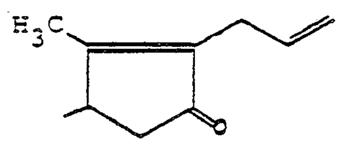

On peut citer également les composés dans lesquels R représente un radical :

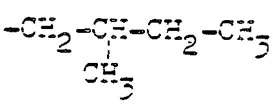 , 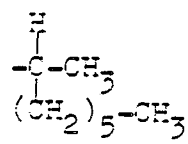 , 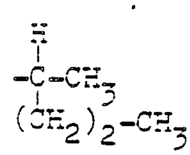 , 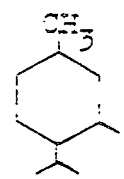

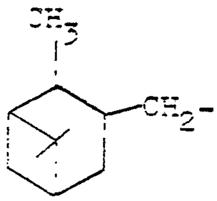 , 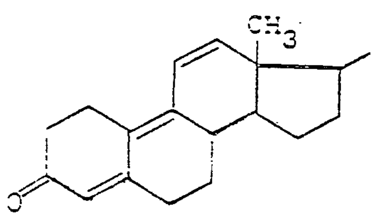 , 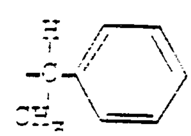 ,

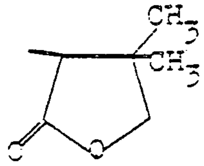

L'invention a naturellement tout particulièrement pour objet les produits de formule I dont la préparation est donnée dans la partie expérimentale.

L'invention a également pour objet un procédé de préparation des composés de formule générale I tels que définis ci-dessus, caractérisé en ce que l'on fait réagir l'hydrure de diisobutyl aluminium au sein d'un solvant organique sur un composé racémique ou optiquement actif de formule générale II :

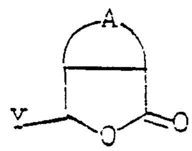

(II)

dans laquelle A et Y conservent les significations précitées, puis soumet le composé de formule générale III résultant :

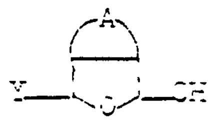

(III)

à l'action d'un alcool racémique ou optiquement actif de formule générale IV :

R—OH (IV)

formule dans laquelle R conserve les significations précitées, pour obtenir le composé de formule I correspondant, dont on sépare, le cas échéant, les diastéréoisomères.

Dans un mode de réalisation préféré, le solvant organique au sein duquel on fait réagir l'hydrure de diisobutyl aluminium avec le composé de formule générale (II) est choisi dans le groupe constitué par les hydrocarbures aliphatiques, aromatiques, les éthers, linéaires ou cycliques.

La réaction des composés III et IV a lieu de préférence en présence d'un acide tel que l'acide paratoluène sulfonique, l'acide méthane sulfonique, l'acide chlorhydrique, l'acide sulfurique, l'acide phosphorique, l'acide métanitrobenzène sulfonique, l'acide 5-sulfosalicyclique et l'acide camphosulfonique, et de préférence au sein d'un solvant organique choisi dans le groupe constitué par les hydrocarbures aliphatiques et aromatiques, les éthers, les hydrocarbures chlorés et les cétones aliphatiques.

L'eau formée lors de la condensation de l'alcool et du composé hémiacétalique peut être éliminée par entraînement azéotropique au reflux d'un solvant choisi dans le groupe constitué par les solvants chlorés, les hydrocarbures aromatiques ou aliphatiques et les éthers.

La séparation des composés I diastéréoisomères est effectuée par cristallisation ou chromatographie.

L'invention a aussi pour objet un procédé tel que défini précédemment, utilisable, lorsque Y représente un atome d'hydrogène, caractérisé en ce que l'on fait réagir un hydroborure alcalin, au sein d'un solvant, puis un acide au sein d'un solvant organique, avec un composé de formule générale V :

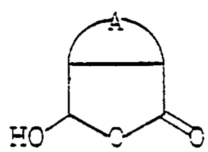

(V)

dans laquelle A conserve la signification précédente, pour obtenir le composé de formule générale ($II_A$) :

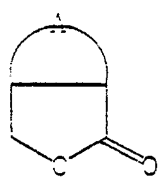

(IIA)

que l'on traite selon le procédé ci-dessus.

Les composés (V) sont décrits dans le brevet français 2 423 488.

Lorsque Y représente $CCl_3$ ou $CBr_3$, les composés II utilisés au départ du procédé de l'invention sont décrits dans le brevet français 2 396 006.

Dans un mode d'exécution préféré de ce procédé particulier le solvant au sein duquel on fait réagir l'hydroborure alcalin est choisi dans le groupe constitué par l'eau, le diméthyl formamide et un alcool aliphatique ; l'acide que l'on fait réagir avec le produit résultant de l'action de l'hydroborure est l'acide paratoluène sulfonique ; le solvant organique au sein duquel on fait réagir l'acide est choisi dans le groupe constitué par le benzène, le xylène et le toluène.

Certains produits de formule III obtenus lors de la mise en œuvre du procédé de l'invention sont connus par les références de la littérature suivantes :

Grandguillot et al., CA 92 (1980) 94159h

Gibson et al., J.A.C.S. 91 (17) 4771-78 (1969)
Bolan et al., Chem. Ber. 110 1823-32 (1977)
Maier et al., Chem. Ber. 101 1354-70 (1968)

Il s'agit de certains produits de formule III dans laquelle Y représente un atome d'hydrogène. D'autres produits de formule III sont nouveaux.

L'invention a donc pour objet ces produits de formule III à titre de produits chimiques nouveaux et, plus particulièrement à titre de produits intermédiaires nécessaires à la mise en œuvre du procédé de l'invention.

Ces produits de formule III nouveaux répondent à la formule générale $III_a$ :

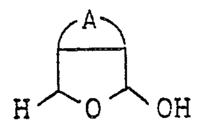

(IIIA)

dans laquelle la chaîne A a pour structure soit :

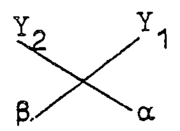

$Y_1$ et $Y_2$, identiques ou différents, représentant un atome de fluor, de chlore ou de brome ou un radical alcoyle comportant de 1 à 6 atomes de carbone, $Y_1$ et $Y_2$ pouvant représenter notamment un radical méthyle ou $Y_1$ et $Y_2$ formant ensemble avec l'atome de carbone auquel ils sont liés, un homocycle carboné comportant de 3 à 7 atomes de carbone,

soit :

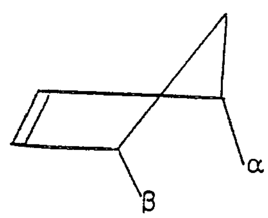

soit

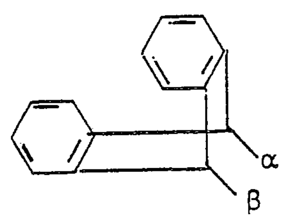

soit :

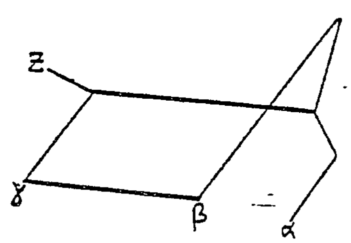

formule dans laquelle Z représente un atome d'hydrogène, un atome de chlore, un atome de brome ou d'iode et à la formule générale $III_b$ :

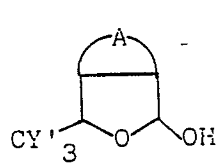

(IIIb)

dans laquelle Y' et A conservent la même signification que précédemment.

L'invention a tout particulièrement pour objet à titre de produits chimiques nouveaux et plus spécialement à titre de produits intermédiaires nécessaires à la mise en œuvre du procédé de l'invention, les produits de formule III dont la préparation est donnée plus loin dans la partie expérimentale.

L'invention a également pour objet l'application des composés de formule I tels que définis précédemment au dédoublement des composés de formule générale III ou IV, application qui consiste à faire réagir :
ou bien un composé hémiacétalique optiquement actif de formule générale III :

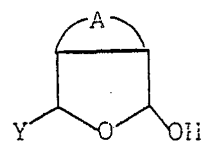

(III)

dans laquelle Y et A conservent leurs significations précitées, avec un alcool racémique de formule générale IV :

R—OH (IV)

dans laquelle R conserve sa signification précédente,
ou bien un composé hémiacétalique racémique de formule générale III :

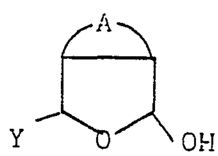

(III)

dans laquelle Y et A conservent leurs significations précédentes avec un alcool optiquement actif de formule générale IV :

R—OH (IV)

dans laquelle R conserve sa signification précédente, pour obtenir un mélange de diastéréoisomères de formule I que l'on sépare selon les procédés classiques, application caractérisée en ce que l'on clive lesdits diastéréoisomères de formule I, pour obtenir respectivement les énantiomères de l'alcool de formule IV ou du composé hémiacétalique de formule III mis en jeu.

Dans un mode de réalisation préféré de l'application selon l'invention,

le clivage des diastéréoisomères est effectué dans un mélange de dioxane et d'eau par l'acide paratoluène sulfonique.

L'invention a plus particulièrement pour objet les applications des composés de formule I décrites dans la partie expérimentale et notamment l'application caractérisée en ce que l'on clive les diastéréoisomères résultants de l'action d'un composé hémiacétalique de formule III et d'un alcool de formule IV choisi dans le groupe constitué par :

l'alcool (RS) α-cyano 3-phénoxy benzylique,

l'alcool (RS) α-cyano 4-fluoro 3-phénoxy benzylique,

l'alcool (RS) cyano 6-phénoxy 2-pyridyl méthylique,

ainsi que l'application caractérisée en ce que l'on clive les diastéréoisomères résultants de l'action d'un composé hémiacétalique de formule III et de la (RS) alléthrolone.

Certains des produits de formule IV ainsi dédoublés sont des produits nouveaux, l'invention a donc pour objet ces produits à titre de produits chimiques nouveaux.

Parmi ces composés, on peut citer tout spécialement les composés de formule IV dont les noms suivent :

l'alcool (S) -cyano(6-phénoxy 2-pyridyl) méthylique,

l'alcool (R) -cyano(6-phénoxy 2-pyridyl) méthylique.

Comme le met clairement en évidence la partie expérimentale, la mise en œuvre des composés de formule I constitue une méthode de dédoublement des composés de formules III et IV tout à fait remarquable. Cette méthode permet en particulier de dédoubler des alcools très fragiles comme, par exemple, les cyanhydrines. Il a été ainsi possible de dédoubler certaines cyanhydrines non encore dédoublées jusqu'ici, comme, par exemple, les alcools de formule :

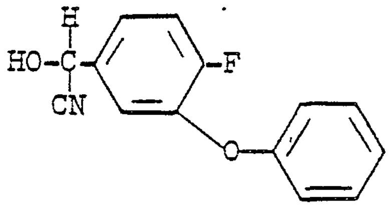

et

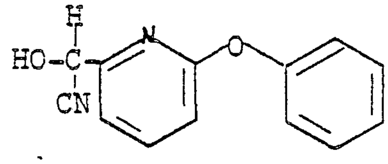

Le procédé de dédoublement des alcools peut se schématiser comme suit :

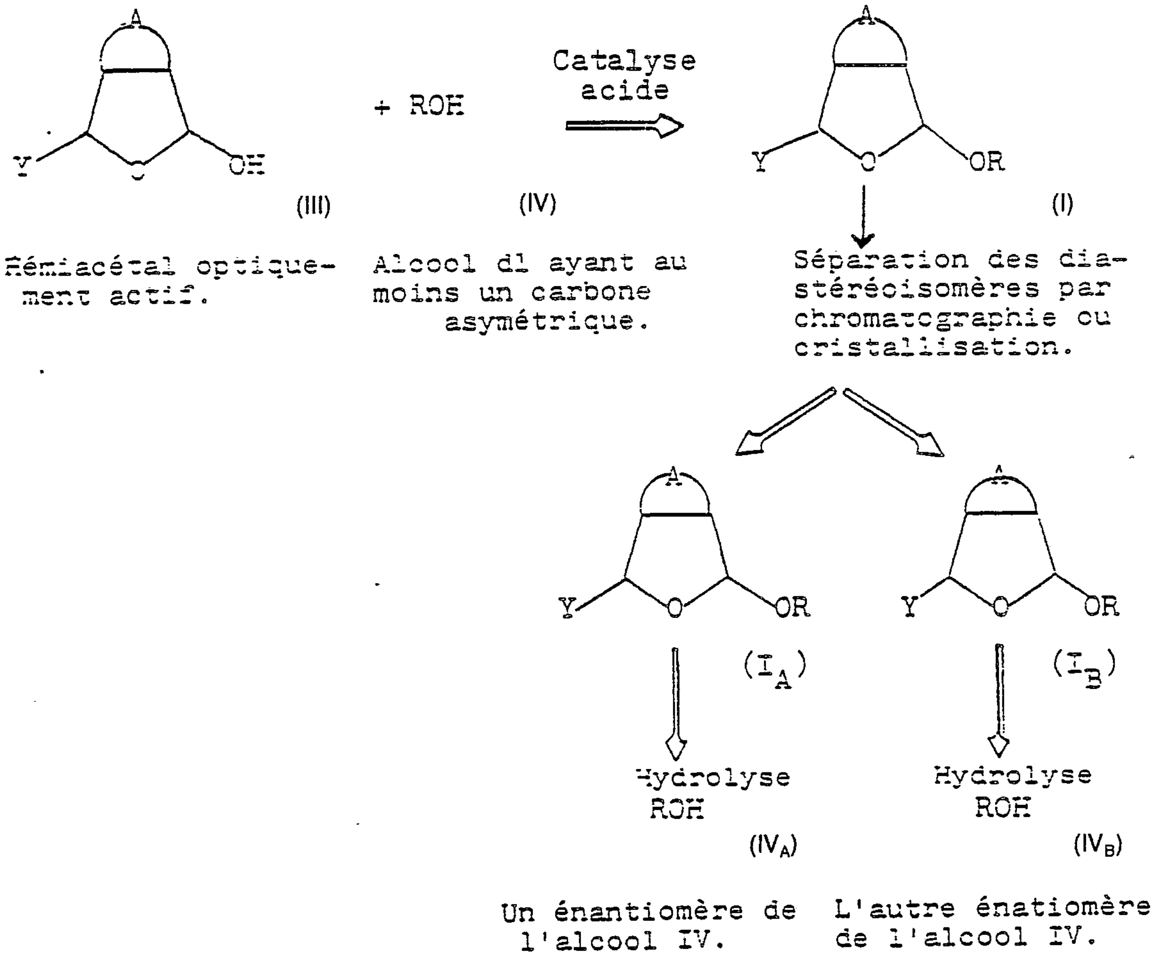


Le procédé du dédoublement des produits de formule III peut naturellement être schématisé de façon analogue.

Cette méthode n'est pas limitée au dédoublement de produits de formule III ou IV particuliers, elle est au contraire très générale, elle permet le dédoublement de très nombreux produits avec d'excellents rendements en metant en œuvre des réactions simples dans des conditions douces.

Les exemples suivants illustrent l'invention sans toutefois la limiter.

Exemple 1 : (1R,2R,5S) 6,6-diméthyl 3-oxa 2-[(S) cyano(3-phénoxy phényl) méthoxy] bicyclo(3.1.0) hexane produit A, et (1R,2R,5S) 6,6-diméthyl 3-oxa 2-[(R) cyano(3-phénoxyphényl) méthoxy] bicyclo(3.1.0) hexane produit B.

On mélange 410 mg d'alcool (RS) α-cyano 3-phénoxy benzylique, 256 mg de (1R,2S,5S) 6,6-diméthyl 3-oxa bicyclo(3.1.0) hexane 2-ol, 10 $cm^3$ de chlorure de méthylène, 15 mg d'acide paratoluène sulfonique, porte au reflux, l'y maintient pendant 30 minutes, refroidit. On concentre à sec par distillation sous pression réduite, chromatographie le résidu sur silice en éluant par du benzène, et obtient 461 mg de (1R,2R,5S) 6,6-diméthyl 3-oxa 2-[RS cyano(3-phénoxyphényl) méthoxy] bicyclo(3.1.0) hexane.

Isolement du (1R,2R,5S) 6,6-diméthyl 3-oxa 2-[S α-cyano(3-phénoxyphényl) méthoxy] bicyclo(3.1.0) hexane.

Au produit isolé ci-dessus, on ajoute 2,5 $cm^3$ d'isopropanol, 0,2 $cm^3$ d'eau, 1 goutte de triéthylamine,

quelques cristaux de produit (S) obtenus par chromatographie, agite pendant 17 heures à température ambiante, agite à 0 + 5 °C, isole par essorage le précipité formé et obtient 105 mg de (1R,2R,5S) 6,6-diméthyl 3-oxa 2-[(S) cyano(3-phénoxyphényl) méthoxy] bicyclo(3.1.0) hexane. F = 70 °C, $(\alpha)_D = 78° \pm 1,5°$ (c = 1 % benzène).

Dichroïsme circulaire :
— max à 263 nm $\Delta\varepsilon = +0,10$
— max à 275 nm $\Delta\varepsilon = -0,19$
— max à 287 nm $\Delta\varepsilon = +0,10$
Spectre de RMN (deutérochloroforme) :
— pic à 1 ppm attribué aux hydrogènes des méthyles géminés,
— pics à 1,42-1,67 ppm attribués aux hydrogènes du cyclopropyle,
— pics à 3,7-4,7 ppm attribués à un hydrogène en position 4α du noyau lactol,
— pics à 4,1-4,2 ppm attribués à l'autre hydrogène en position 4β du noyau lactol,
— pics à 4,9-5,3 ppm attribués à un hydrogène en position 2 du noyau lactol et à l'hydrogène porté par le même carbone que le groupement CN,
— pics à 6,8-7,5 ppm attribués aux hydrogènes des noyaux aromatiques.

Isolement du (1R,2R,5S) 6,6-diméthyl 3-oxa 2-[R-cyano(3-phénoxyphényl) méthoxy] bicyclo(3.1.0) hexane.

Par chromatographie sur silice du produit isolé au début de l'exemple, en éluant par du benzène, on obtient le produit B dont les caractéristiques sont les suivants :

Dichroïsme circulaire (dioxane) :
— max vers 235 nm $\Delta\varepsilon = +3$
— max à 277 nm $\Delta\varepsilon = -0,1$
— max à 285 nm $\Delta\varepsilon = -0,2$
Spectre de RMN (deutérochloroforme) :
— pic à 1,04 ppm attribué aux hydrogènes des méthyles géminés,
— pics à 1,48-1,7 ppm attribués aux hydrogènes de l'isopropyl,
— pics à 3,9-4,1 ppm attribués aux hydrogènes en position 4 du cycle hémiacétalique,
— pics à 5,2-5,5 ppm attribués à l'hydrogène en position 2 du cycle hémiacétalique, et à l'hydrogène porté par le même carbone que le groupement CN,
— pics à 6,8-8,1 ppm attribués aux hydrogènes aromatiques.

Préparation du produit de départ :

Le (1R,2S,5S) 6,6-diméthyl 3-oxa bicyclo [3.1.0] hexane 2-ol utilisé comme produit de départ a été préparé comme suit :

Stade A : (1R,5S) 6,6-diméthyl 3-oxa bicyclo(3.1.0) hexane-2-one.

Dans 800 $cm^3$ d'eau on introduit 170,6 g de (1R,5S) 6,6-diméthyl 4(R)-hydroxy-3-oxa bicyclo(3.1.0) hexane-2-one, ajoute lentement à 0 °C une solution de 127,2 g de carbonate de sodium dans 400 $cm^3$ d'eau, agite pendant 15 minutes à 0 °C, ajoute lentement par petites fractions 45,4 g de borohydrure de sodium agite pendant 4 heures à 0 °C, acidifie à pH 2 par addition d'une solution aqueuse 2N d'acide chlorhydrique, sature par addition de chlorure de sodium, laisse une nuit au repos, extrait au chlorure de méthylène, concentre à sec par distillation sous pression réduite, rectifie le résidu sous pression réduite et obtient 130,2 g du produit recherché.

$(\alpha)_D = -92°$ (c = 1 % éthanol).

Stade B : (1R,2S,5S) 6,6-diméthyl 3-oxa bicyclo(3.1.0) hexane-2-ol.

Dans 720 $cm^3$ de toluène, on introduit 60 g de (1R,5S) 6,6-diméthyl 3-oxa bicyclo(3.1.0) hexane-2-one, ajoute lentement à —70 °C, 404 $cm^3$ de solution toluénique 1,2 M d'hydrure de diisobutyl aluminium, agite pendant 16 minutes à —65 °C, verse lentement le mélange réactionnel dans une solution aqueuse de tartrate double de sodium et de potassium, agite, laisse au repos, sépare par décantation la phase organique, extrait la phase aqueuse par de l'éther éthylique, réduit les phases organiques, ajoute du chlorure de méthylène, concentre à sec par distillation sous pression réduite et obtient 56,7 g du produit recherché.

$(\alpha)_D = +66,5°$ (c = 1 % éthanol) Eb ≈ 50 °C sous 0,5 mm de mercure.

Exemple 2 : (1R,2R,4R,5S) 6,6-diméthyl 3-oxa 2-[(S) cyano(3-phénoxy phényl) méthoxy] 4-(2-méthyl 2-propyl) bicyclo(3.1.0) hexane (produit A) et (1R,2R,4R,5S) 6,6-diméthyl 3-oxa 2-[(R) cyano (3-phénoxy phényl) méthoxy] 4-(2-méthyl 2-propyl) bicyclo(3.1.0) hexane (produit B).

On mélange 2 g de (1R,2S,4R,5S) 6,6-diméthyl 4-[(2-méthyl 2-propyl] 3-oxa bicyclo(3.1.0) hexan-2-ol,

30 $cm^3$ de benzène, 2,5 g d'alcool (RS) α-cyano 3-phénoxy benzylique, 10 mg d'acide paratoluène sulfonique, agite à 20 °C pendant 20 heures, lave le mélange réactionnel par une solution aqueuse de bicarbonate de sodium, concentre à sec sous pression réduite et chromatographie le résidu sur gel de silice en éluant par un mélange d'éther de pétrole (Eb. 35-70 °C) et d'éther éthylique (9/1) et l'on obtient 1,15 g de produit A et 0,600 g de produit B.

Les produits A et B possèdent les caractéristiques suivantes :

Produit A : Spectre de RMN (deutérochloroforme) :
— pic à 0,85 ppm attribué aux hydrogènes des méthyles du terbutyle,
— pic à 1,06 ppm attribué aux méthyles géminés,
— pics de 1,25 à 1,72 ppm attribués aux hydrogènes du cyclopropyle,
— pic à 3,6 ppm attribué à l'hydrogène de O—CH—tBu,
— pics à 5,2-5,4 ppm attribués aux hydrogènes de O—CH—O et de CH—CN.

Produit B : Spectre de RMN (deutérochloroforme) :
— pic à 1,025 ppm attribué aux hydrogènes des méthyles du terbutyle,
— pics à 0,97-1,07 ppm attribués aux méthyles géminés,
— pics à 1,33-1,45 ppm et 1,53-1,65 ppm attribués aux hydrogènes du cyclopropyle ;
— pic à 3,6 ppm attribué à l'hydrogène de O—CH—tBu,
— pics à 4,8-5,4 ppm attribués aux hydrogènes de O—CH—O et de CH—CN.

Préparation du produit de départ :

le (1R,2R,4R,5S) 6,6-diméthyl 4-(2-méthyl 2-propyl) 3-oxa bicyclo(3.1.0) hexane 2-ol utilisé comme produit de départ, peut être préparé comme suit :

Stade A : Acide 1R,cis 2,2-diméthyl 3-[1-hydroxy 2,2-diméthyl propyl] cyclopropane carboxylique.

Dans 50 $cm^3$ de tétrahydrofuran, on introduit 5 g de 1R,cis 6,6-diméthyl 3-oxa 4R-hydroxy bicyclo(3.1.0) hexane-2-one, ajoute lentement à —70 °C 46 $cm^3$ de solution 1,5 M de terbutyl lithium dans le pentane, agite pendant 1 heure, verse le mélange réactionnel dans une solution aqueuse de phosphate monosodique, extrait au chlorure de méthylène, concentre à sec par distillation sous pression réduite et chromatographie le résidu sur gel de silice en éluant par un mélange de benzène et d'acétate d'éthyle (1/1) et obtient le produit recherché. F = 129 °C.

Stade B : (1R,4R,5S) 6,6-diméthyl 4-[2-méthyl prop-2-yl] 3-oxa bicyclo(3.1.0) hexan-2-one.

Dans 50 $cm^3$ de benzène, on dissout 5 g d'acide 1R,cis 2,2-diméthyl 3-[1-hydroxy 2,2-diméthyl propyl] cyclopropane carboxylique, porte le mélange réactionnel au reflux, ajoute 20 mg d'acide paratoluène sulfonique, maintient au reflux pendant un quart d'heure, refroidit, lave le mélange réactionnel par une solution aqueuse de soude, concentre à sec sous pression réduite, purifie le résidu par chromatographie sur gel de silice en éluant par le mélange benzène-acétate d'éthyle (9/1), et obtient le produit recherché. F = 113 °C.

Stade C : (1R,2R,4R,5S) 6,6-diméthyl 4-(2-méthyl prop-2-yl) 3-oxa bicyclo(3.1.0) hexan-2-ol.

Dans 25 $cm^3$ de toluène, on introduit 2,5 g de (1R,4R,5S) 6,6-diméthyl 4-(2-méthyl prop-2-yl) 3-oxa bicyclo(3.1.0) hexan-2-one, ajoute lentement à —70 °C, 15,5 $cm^3$ de solution toluénique à 20 % d'hydrure de diisobutyl aluminium, agite, verse le mélange réactionnel sur de la glace, élimine par filtration l'insoluble formé, extrait à l'éther éthylique, concentre à sec par distillation sous pression réduite et obtient 2,5 g de produit recherché. F = 126 °C.

Exemple 3 : (1R,2S,4R,5S) 6,6-diméthyl 4-trichlorométhyl 2-[1(R) 2-méthyl 4-oxo 3-(2-propen-1-yl) cyclopent-2-enyloxy] 3-oxa bicyclo(3.1.0) hexane (produit A) et (1R,2S,4R,5S) 6,6-diméthyl 4-trichlorométhyl 2-[(1S) 2-méthyl 4-oxo 3-(2-propen-1-yl) cyclopent-2-enyloxy] 3-oxa bicyclo(3.1.0) hexane (produit B).

Dans 40 $cm^3$ de chlorure de méthylène, on introduit 3,21 g de RS alléthrolone, 40 mg d'acide paratoluène sulfonique, 0,800 g d'(1R,2S,4R,5S) 6,6-diméthyl 4-(trichlorométhyl) 3-oxa bicyclo(3.1.0) hexan-2-ol, porte le mélange réactionnel au reflux dans un appareil de Dean-Stark garni de gel d'aluminium anhydre, ajoute en 4 fois la même quantité de lactol, toutes les demi-heures (soit 4 g de lactol au total), laisse en tout 4 heures au reflux, lave le mélange réactionnel par une solution aqueuse de bicarbonate de sodium, à l'eau, concentre la phase organique à sec par distillation sous pression réduite, ajoute de l'eau, isole par essorage les cristaux formés, ajoute de l'acétone, élimine par filtration l'insoluble résiduel, concentre le filtrat acétonique à sec, ajoute du chlorure de méthylène, lave la solution organique à l'eau, concentre à sec par distillation sous pression réduite, ajoute de l'éther isopropylique, à 50 °C, laisse une nuit à 20 °C, refroidit à 0 °C, isole par essorage le précipité formé, le sèche, le dissout dans le chlorure de méthylène, filtre, concentre le filtrat à sec sous pression réduite, ajoute de l'éther isopropylique à 50 °C, laisse une heure à 20 °C, isole par essorage le précipité formé et obtient 1,82 g de

produit A. F = 128°-130 °C.

Les liqueurs-mères de cristallisation ainsi que les liqueurs-mères de recristallisation sont concentrées à sec par distillation sous pression réduite, le résidu est cristallisé dans l'éther isopropylique et l'on obtient 2,67 g de produit B.

Préparation du produit de départ :

Le (1R,2S,4R,5S) 6,6-diméthyl 4-(trichlorométhyl) 3-oxa bicyclo(3.1.0) hexan-2-ol utilisé comme produit de départ, peut être préparé comme suit :

Dans 200 $cm^3$ de toluène, on dissout 20 g de (1R,2S,4R,5S) 6,6-diméthyl 4-(trichlorométhyl) 3-oxa bicyclo(3.1.0) hexan-2-one, refroidit à — 60 °C, ajoute lentement 75 $cm^3$ de solution à 20 % d'hydrure de diisobutyl aluminium ; agite pendant 1 heure à — 60 °C, verse le mélange réactionnel sur un mélange d'eau et de glace, ajoute une solution aqueuse N d'acide chlorhydrique jusqu'à pH 3-4, filtre, sépare par décantation la phase benzénique, la lave par une solution aqueuse de bicarbonate de sodium à l'eau, concentre à sec sous pression réduite, ajoute de l'éther de pétrole (Eb. 35-70 °C) et obtient 14,5 g de produit attendu. F = 140 °C.

Exemple 4 : (1R,2S,4R,5S) 6,6-diméthyl 4-tribromométhyl 2-[1(R) 2-méthyl 4-oxo 3-(2-propen-1-yl) cyclopent-2-ényloxy] 3-oxa bicyclo(3.1.0) hexane.

Dans 20 $cm^3$ de chlorure de méthylène, on introduit 0,8 g de (RS) alléthrolone, 2 g de (1R,2S,4R,5S) 6,6-diméthyl 4-tribromométhyl 3-oxa(3.1.0) bicyclo hexan-2-ol, 20 mg d'acide paratoluène sulfonique, porte le mélange réactionnel au reflux, maintient le reflux pendant 4 heures, laisse 16 heures à 20 °C, élimine par filtration l'insoluble formé, concentre le filtrat à sec par distillation sous pression réduite, ajoute au résidu un mélange d'éther de pétrole (Eb. 35-70 °C) et d'éther éthylique (8/2), élimine par filtration l'insoluble formé, concentre le filtrat à sec par distillation sous pression réduite, ajoute au résidu de l'éther de pétrole (Eb. 35-70 °C), isole par essorage le précipité formé et obtient 0,726 g de (1R,2S,4R,5S) 6,6-diméthyl 4-tribromométhyl 2-[1(R) 2-méthyl 4-oxo 3-(2-propen-1-yl) cyclopent-2-enyloxy] 3-oxa bicyclo(3.1.0) hexane. F = 126-128 °C. $(\alpha)_D = + 37,5°$ (c = 1 % benzène).

Préparation du produit de départ : Le (1R,5S) 6,6-diméthyl 4(R)-tribromométhyl-3-oxa(3.1.0) bicyclo hexan-2(S)-ol utilisé comme produit de départ, peut être préparé comme suit :

Dans 200 $cm^3$ de toluène, on introduit 20 g de (1R,5S) 6,6-diméthyl 4(R) tribromométhyl-3-oxa bicyclo(3.1.0) hexan-2-one, ajoute lentement à — 60 °C 50 $cm^3$ de solution à 20 % dans le toluène d'hydrure de diisobutyl aluminium, agite pendant 1 heure à — 60 °C, verse le mélange réactionnel sur un mélange de glace et de solution aqueuse normale d'acide chlorhydrique, amène à 20 °C, élimine par filtration l'insoluble formé, sépare par décantation la phase benzénique, la lave par une solution aqueuse de bicarbonate de sodium, à l'eau, concentre à sec sous pression réduite et obtient 17,6 g de produit recherché.

Exemple 5 : (3aR,4R,7S,7aS,1R) 1-[R-(cyano-3-phénoxy phényl méthoxy] tétrahydro 4,7-méthano isobenzofurane (produit A) et (3aR,4R,7S,7aS,1R)-1-[S(cyano-3-phénoxy phényl) méthoxy] tétrahydro 4,7-méthano isobenzofurane (produit B).

Dans 70 $cm^3$ de chlorure de méthylène, on introduit 8,1 g d'alcool (RS) α-cyano-3-phénoxy benzylique, 80 mg d'acide paratoluène sulfonique, ajoute par petites fractions sous agitation 4,2 g de (3aR,4R,7S,7aS,1S) tétrahydro 4,7-méthano isobenzofuran 1-ol (F = 200 °C) agite pendant 15 minutes, concentre à sec par distillation sous pression réduite, chromatographie sur gel de silice en éluant par un mélange de benzène et d'acétate d'éthyle (95/5) chromatographie à nouveau sur gel de silice en éluant avec un mélange d'éther de pétrole (Eb. 40-70 °C) et d'éther éthylique (75/25) et obtient 4 g de produit A, et 3,5 g de produit B.

Les caractéristiques des isomères A et B sont les suivantes :

Isomère A :

Spectre de RMN (deutérochloroforme).

— pics de 3,4 à 4 ppm attribués aux hydrogènes de $—OCH_2$,

— pics à 5,0-5,4 ppm attribués à l'hydrogène de

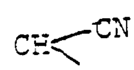

— pic à 6,2 ppm attribué aux hydrogènes éthyléniques,

— pics de 6,8 à 7,5 ppm attribués aux hydrogènes des noyaux aromatiques.

Isomère B :

Spectre de RMN (deutérochloroforme).
— pics à 3,5-3,6 ppm attribués aux hydrogènes de $CH_2O$
— pic à 5,2 ppm attribué à l'hydrogène de

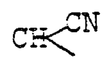

— pics de 5,9 à 6,3 ppm attribués aux hydrogènes éthyléniques,
— pics de 6,8 à 7,5 ppm attribués aux hydrogènes des noyaux aromatiques.

Préparation du produit de départ : Le (3aR,4R,7S,7aS,1S) tétrahydro 4,7-méthano isobenzofuran-1-ol utilisé comme produit de départ, a été préparé comme suit :

Stade A : 1-hydroxy 3-oxo 1,3,3a,7a-tétrahydro 4,7-méthano isobenzofurane (lactone racémique).

Dans 1,05 litre de chloroforme, on introduit 208 g de 5-hydroxy 2(5H) furanone, 350 mg d'hydroquinone, ajoute lentement 244 $cm^3$ de cyclopentadiène, maintient la température à 45-47 °C, agite pendant 17 heures à 20 °C, concentre à sec sous pression réduite, ajoute de l'éther isopropylique au résidu, porte au reflux sous agitation, refroidit, isole par essorage le précipité formé, sèche, dissout dans le chlorure de méthylène, ajoute du charbon actif, concentre à sec par distillation sous pression réduite, porte au reflux sous agitation dans l'éther isopropylique, refroidit, isole par essorage, le précipité formé et obtient 325,7 g de produit recherché (racémique). F = 105 °C.

Stade B : (3R,3aR,4R,7S,7aS) 3-[(1S) 2-méthyl-4-oxo 3-(propen-1-yl) cyclopent-2-enyloxy] tétrahydro 4,7 méthano isobenzofuran-1-one.

Dans 350 $cm^3$ de benzène, on introduit 54 g de (S) alléthrolone, 55,7 g de 1-hydroxy 3-oxo-tétrahydro 1,3,3a,7a,4,7 méthano isobenzofurane (racémique) obtenue au stade A précédent, 6,75 g d'acide paratoluène sulfonique porte le mélange réactionnel au reflux, concentre à sec par distillation sous pression réduite, ajoute de l'éther isopropylique au résidu, isole par essorage les cristaux formés et obtient 36,5 g de produit recherché. F = 148 °C, $(\alpha)_D = -8{,}5°$ (c = 1 % benzène).

Par chromatographie des liqueurs-mères on obtient un 2[e] jet de 12,3 g du même produit. F = 145 °C.

Stade C : (3R,3aR,4R,7S,7aS) 3-hydroxy tétrahydro 4,7 méthano isobenzofuran-1-one.

Dans un mélange de 188 $cm^3$ de dioxane et 355 $cm^3$ d'eau on introduit 35,5 g de (3R,3aR,4R,7S,7aS) 3-[(1S) 2-méthyl 4-oxo 3-(propen-1-yl) cyclopent-2-ényloxy] tétrahydro 4,7 méthano isobenzofuran-1-one obtenu au stade B précédent, 3,5 g d'acide paratoluène sulfonique, porte le mélange réactionnel au reflux maintient le reflux pendant 2 heures, neutralise à ≃ pH 7 la solution obtenue avec 4 $cm^3$ de triéthylamine, concentre à sec sous pression réduite, isole par essorage les cristaux formés et obtient 8,5 g de produit recherché. pF = 120°.

Stade D : (3aR,4R,7S,7aS) tétrahydro 4,7 méthano isobenzofuran-1-one.

Dans 30 $cm^3$ d'eau on introduit 3 g de (3R,3aR,4S,7R,7aS) 3-hydroxy tétrahydro 4,7-méthano isobenzofuran-1-one obtenu au stade précédent, refroidit à 0 °C, ajoute lentement 0,7 g d'hydroborure de sodium, agite jusqu'à transformation complète du produit de départ, acidifie à pH 2 par une solution d'acide chlorhydrique N, extrait au chlorure de méthylène, concentre la phase chlorométhylénique à sec par distillation sous pression réduite, ajoute du benzène et 50 mg d'acide paratoluène sulfonique, porte le mélange réactionnel à 30 °C, l'y maintient pendant 30 minutes, concentre à sec par distillation sous pression réduite, cristallise le résidu dans l'éther isopropylique et obtient 1,9 g de produit recherché. F = 131 °C.

Stade E : (3aR,4R,7S,7aS,1S) tétrahydro 4,7 méthano isobenzofuran-1-ol.

Dans 50 $cm^3$ de toluène on introduit 5,6 g de (3aR,4R,7S,7aS) tétrahydro 4,7 méthano isobenzofuran-1-one obtenu au stade D précédent, refroidit à —70 °C ajoute lentement 33 $cm^3$ de solution 1,2 M d'hydrure de diisobutyl aluminium dans le toluène, on verse le mélange réactionnel sur de la glace, agite avec une solution molaire de tartrate double de sodium et de potassium, sépare par décantation la phase aqueuse, extrait au chlorure de méthylène en relargant au chlorure de sodium, concentre à sec les phases organiques réunies par distillation sous pression réduite, chromatographie le résidu sur gel de silice en éluant par un mélange d'éther de pétrole (Eb. 30-75 °C) et d'acétate d'éthyle (1/1) et obtient 5,3 g de produit recherché.

F = 100 °C, $(\alpha)_D = +53{,}5°$ (c = 1 % benzène).

Exemple 6 : (11R,12S,15S) 12-[(S) cyano(3-phénoxyphényl) méthoxy] 9,10,11,12,14,15-hexahydro 9,10 [3',4'] furano anthracène (produit A) et (11S,12R,15R) 12-[(S)-cyano(3-phénoxy phényl) méthoxy] 9,10,11,12,14,15-hexahydro 9,10 [3',4'] furano anthracène (produit B).

Dans 100 $cm^3$ de chlorure de méthylène on introduit 12,5 g d'alcool (S) α-cyano-3-phénoxy benzylique, 100 mg d'acide paratoluène sulfonique, porte le mélange réactionnel au reflux, ajoute lentement par petites fractions 8 g de 9,10,11,12,14,15-hexahydro-9,10 [3',4'] furano anthracène 12-ol racémique (F = 187 °C), maintient le reflux pendant 30 minutes, laisse refroidir, lave à l'eau et concentre à sec par distillation sous pression réduite. On ajoute au résidu obtenu de l'éther isopropylique, agite pendant 17 heures, isole par essorage les cristaux formés. On concentre à sec les liqueurs-mères, ajoute un mélange de méthanol et d'eau (2/1), extrait à l'heptane, joint la phase heptanique aux cristaux précédemment isolés, chromatographie l'ensemble sur gel de silice en éluant par du benzène et obtient 6,8 g de produit A.

F = 178 °C, $(\alpha)_D$ = — 119° (c = 1 % tétrachlorure de carbone) et 6,1 g de produit B.

F = 167 °C $(\alpha)_D$ = + 111° (c = 1 % $CCl_4$).

Le 9,10,11,12,14,15-hexahydro 9,10 [3',4'] furano anthracène 12-ol racémique. (F = 187 °C) utilisé comme produit de départ peut être préparé comme suit :

Stade A : 9,10,14,15-tétrahydro 9,10 [3',4'] furano anthracène 12(11H) one racémique.

On chauffe à 170 °C un mélange de 1 g d'anthracène et de 1,5 g 2(5H) furanone, en agitant pendant 17 heures, refroidit, ajoute du chlorure de méthylène, ajoute du charbon actif, filtre, concentre à sec par distillation sous pression réduite, chromatographie le résidu sur gel de silice en éluant avec un mélange de benzène et d'acétate d'éthyle (95/5) et obtient 1,2 de produit recherché. F = 231 °C.

Stade B : 9,10,11,12,14,15-hexahydro 9,10 [3',4'] furano anthracène-12-ol racémique.

Dans 70 $cm^3$ de tétrahydrofurane on introduit 5 g de 9,10,14,15-tétrahydro 9,10 [3',4'] furano anthracène 12(11H) one, ajoute lentement 18 $cm^3$ de solution 1,2 molaire d'hydrure de diisobutyl lithium dans le toluène, verse le mélange, réactionne sur un mélange de glace et de solution aqueuse normale d'acide chlorhydrique, extrait au benzène, lave à l'eau, concentre à sec par distillation sous pression réduite, cristallise le résidu dans l'éther isopropylique et obtient 4,7 g de 9,10,11,12,14,15-hexahydro 9,10 [3',4'] furano anthracène-12-ol racémique. F = 187 °C.

Exemple 7 : (11R,12S,15S) 12-[(R) (4,4-diméthyl 2-oxo tétrahydrofuran-3-yl) oxy] 9,10,11,12,14,15-hexahydro-9,10 [3',4'] furano anthracène (produit A) et isomère correspondant (produit B).

On chauffe au reflux, 1,8 g de 4,4-diméthyl 2-oxo 3-hydroxy tétrahydrofuran racémique, 20 $cm^3$ de chlorure de méthylène et 180 mg d'acide paratoluène sulfonique. On ajoute lentement 2,5 g de (11R,12R,15S) 9,10,11,12,14,15-hexahydro 9,10 [3',4'] furano anthracène-12-ol, dissous dans 25 $cm^3$ de chlorure de méthylène. On agite au reflux pendant une heure. On laisse refroidir à la température ambiante, lave au bicarbonate de sodium et à l'eau. On sèche et évapore à sec sous pression réduite. On chromatographie le mélange obtenu sur silice en éluant par le mélange toluène-acétate d'éthyle (9/1). On obtient 1,7 g de produit A fondant à 216 °C. $(\alpha)_D$ = — 115° ± 2,5 (c = 1 % diméthylformamide) ; et 1,55 g de produit B fondant à 270 °C. $(\alpha)_D$ = — 89,5° ± 3 (c = 0,5 % diméthylformamide).

Préparation du produit de départ :
(11R,12R,15S) 9,10,11,12,14,15-hexahydro 9,10 [3',4'] furano anthracène-12-ol.

Stade A : (11R,12S,15S) 12-[(1R,2S,5R) 2-prop-2-yl 5-méthyl cyclohexyloxy] 9,10,11,12,14,15-hexahydro-9,10 [3',4'] furano anthracène (produit A) et isomère (11S,12R,15R) correspondant (produit B).

On porte au reflux une solution renfermant 4,2 g de 1-menthol, 100 $cm^3$ de chlorure de méthylène et 100 mg d'acide paratoluène sulfonique. On ajoute lentement 5 g de 9,10,11,12,14,15-hexahydro 9,10 [3',4'] furano anthracène-12-ol racémique. On laisse refroidir à la température ambiante, dilue à l'eau et extrait au chlorure de méthylène. On lave à l'aide d'une solution de bicarbonate de sodium, sèche et évapore à sec sous pression réduite. On chromatographie sur silice en éluant par le mélange benzène-éther de pétrole (Eb. 40-70 °C) (7/3). On isole ainsi 2,9 g de produit A fondant à 180 °C. $(\alpha)_D$ = — 144° (c = 1 % DMF) et 2,5 g de produit B fondant à 170 °C. $(\alpha)_D$ = + 50° (c = 1 % DMF).

Stade B : (11R,12R,15S) 9,10,11,12,14,15-hexahydro 9,10 [3',4'] furano anthracène-12-ol.

On chauffe au reflux 2,5 g du produit B préparé au stade précédent, 30 $cm^3$ de dioxane, 20 $cm^3$ d'eau et 250 mg d'acide paratoluène sulfonique. On refroidit à la température ambiante, dilue à l'eau, extrait au chlorure de méthylène, lave avec une solution saturée de chlorure de sodium puis à l'eau, sèche et

évapore à sec sous pression réduite. On chromatographie sur silice en éluant avec un mélange cyclohexane-acétate d'éthyle (7/3) puis un mélange (1/1) et isole 1,5 g du produit recherché fondant à 160 °C. $(\alpha)_D = -44°$ (c = 0,9 % DMF).

Exemple 8 : (11S,12R,15R) 12-[1R(cyano 3-phénoxy phényl) méthoxy] 9,10 [3',4'] furano anthracène (produit A) et isomère 1S correspondant (produit B).

On chauffe au reflux un mélange renfermant 1,5 g d'alcool RS α-cyano 3-phénoxy benzylique, 30 cm³ de chlorure de méthylène anhydre, 150 mg d'acide paratoluène sulfonique. On ajoute 1,3 g de (11S,12S,15R) 9,10,11,12,14,15-hexahydro 9,10 [3',4'] furano anthracène-12-ol dans 20 cm³ de chlorure de méthylène. On refroidit à la température ambiante, lave avec une solution aqueuse de bicarbonate de sodium, puis à l'eau, sèche et évapore sous pression réduite. On chromatographie sur silice le mélange de produits obtenus en éluant avec du benzène. On obtient ainsi 1,05 g du produit A fondant à 178 °C. $(\alpha)_D = +122°$ (c = 1 % $CCl_4$) et 960 mg de produit B fondant à 167 °C. $(\alpha)_D = +111°$ (c = 1 % $CCl_4$).

Préparation du produit de départ :
(11S,12S,15R) 9,10,11,12,14,15-hexahydro 9,10 [3',4'] furano anthracène-12-ol.

On chauffe au reflux 2,8 g de (11S,12R,15R) 12-[(1R,2S,5R) 2-prop-2-yl 5-méthyl cyclohexyl oxy] 9,10,11,12,14,15-hexahydro-9,10 [3',4'] furano anthracène, préparé à l'exemple 7. F = 180 °C $(\alpha)_D = -144°$ (c = 1 % DMF), 40 cm³ de dioxanne et 20 cm³ d'eau et 300 mg d'acide paratoluène sulfonique. On laisse refroidir à la température ambiante, dilue à l'eau extrait au chlorure de méthylène, lave à l'aide d'une solution saturée de bicarbonate de sodium, puis à l'eau. On sèche et évapore à sec sous pression réduite. On chromatographie sur silice le résidu obtenu, élue par un mélange cyclohexane-acétate d'éthyle (7/3) puis un mélange (1/1). On obtient ainsi 1,7 g du produit recherché. F = 160 °C $(\alpha)_D = +41°$ (c = 1 % DMF).

Exemple 9 : (6-bromohexahydro-3,5-méthano-2H-cyclopenta [b] furan-2-yl) oxy (S) α-cyano-3-phénoxy benzyle (isomère 2R et isomère 2S).

On porte au reflux sous agitation, une solution renfermant 8,79 g d'alcool (S) α-cyano 3-phénoxy benzylique, 200 cm³ de chlorure de méthylène et 880 mg d'acide paratoluène sulfonique. On ajoute en 1 heure 30 minutes, 6,57 g de 6-bromohexahydro-3,5-méthano-2H-cyclopenta [b] furan-2-ol racémique en solution dans 150 cm³ de méthylène. On agite pendant 15 minutes, le mélange réactionnel au reflux, laisse revenir à la température ambiante, décante et lave à l'eau. On sèche la phase organique sur sulfate de sodium, essore, rince et distille à la fin du solvant. On obtient 14,3 g du produit recherché.

On chromatographie le produit obtenu sur silice en éluant par le mélange hexane-acétate d'éthyle 8-2, puis par le mélange hexane-acétate d'éthyle 9-1. On obtient ainsi 7,8 g de 2R(6-bromohexahydro 3,5-méthano 2H-cylopenta [b] furan-2-yl) oxy (S) α-cyano 3-phénoxybenzyle. F = 65 °C $(\alpha)_D = -108°$ (c = 0,5 % DMF) et 3 g de 2S(6-bromo hexahydro 3,5-méthano 2H-cyclopenta [b] furan-2-yl) oxy (S) α-cyano 3-phénoxy benzyle. $(\alpha)_D = +105{,}5°$ (c = 0,5 % DMF).

Le 6-bromo hexahydro 3,5-méthano 2H-cyclopenta [b] furan-2-ol racémique a été obtenu comme suit :

Dans 500 cm³ de toluène on introduit 62,12 g de 6-bromo hexahydro-3,5-méthano-2H-cyclopenta [b] furan-2-one préparé selon la méthode de Ver Nooy el Coll. Am. Soc. 77 3583 (1955), ajoute lentement à — 60 °C 275 cm³ d'une solution toluénique d'hydrure de diisobutyl aluminium à 1,2 mole/l, agite pendant 45 minutes à — 60 °C, verse le mélange réactionnel sec dans une solution aqueuse molaire de tartrate double de sodium et de potassium, agite pendant 2 heures, sépare la phase organique par décantation, sature la phase aqueuse par du chlorure de sodium, extrait au chlorure de méthylène, réunit les phases organiques, les concentre à sec par distillation sous pression réduite et obtient 64,7 g de produit recherché. F = 104 °C .

Exemple 10 : (RS) α-[2S(6-bromo hexahydro 3,5-méthano 2H-cyclopenta [b] furan-2-yl) oxy] 3-phénoxy benzène acétonitrile et isomère (R) α (produit A) et isomère (S) α (produit B).

On porte au reflux 2,93 g d'alcool (RS) α-cyano 3-phénoxy benzylique, 100 cm³ de chlorure de méthylène et 300 mg d'acide paratoluène sulfonique. On introduit une solution de 2,19 g de (2S) 6-bromo hexahydro 3,5-méthano 2H-cyclopenta [b] furan-2-ol dans 50 cm³ de chlorure de méthylène. On agite encore 15 minutes au reflux après la fin de l'introduction. On ramène à la température ambiante, lave, sèche, essore, rince et distille à sec sous pression réduite. On obtient 4,34 g de produit que l'on purifie par chromatographie sur silice en éluant par le mélange hexane-acétate d'éthyle 9-1. On obtient ainsi le produit A fondant à 65 °C $(\alpha)_D = +110°$ (c = 0,5 % DMF) et le produit B $(\alpha)_D = +108°5$ (c = 0,4 % DMF).

Le (2S) 6-bromo hexahydro 3,5-méthano 2H-cyclopenta [b] furan-2-ol a été préparé comme suit :

Stade A : (2S) 6-bromo hexahydro 3,5-méthano 2H-cyclopenta [b] 2-[(3R) (4,4-diméthyl 2-oxo tétrahydrofuran-3-yl) oxy] furan.

On porte au reflux un mélange de 46,33 g de D(—) 2-oxo 4,4-diméthyl 3-hydroxy tétrahydrofuran, 460 $cm^3$ de chlorure de méthylène et 460 mg d'acide paratoluène sulfonique. On ajoute 60 g de 6-bromo hexahydro 3,5-méthano 2H-cyclopenta [b] furan-2-ol racémique dissous dans 360 $cm^3$ de chlorure de méthylène. On agite 2 heures au reflux en éliminant l'eau formée, ramène à la température ambiante, décante, lave, sèche, essore et distille à sec sous pression réduite. On obtient 94,7 g de produit que l'on chromatographie sur silice en éluant par le mélange hexane-acétate d'éthyle (8-2). On obtient ainsi le produit A fondant à 178 °C $(\alpha)_D = +156°$ (c = 1 % DMF) et le produit B fondant à 104 °C $(\alpha)_D = -122°$ (c = 1 % DMF).

Stade B : (2S) 6-bromo hexahydro 3,5-méthano 2H-cyclopenta [b] furan-2-ol.

On porte au reflux un mélange renfermant 4,2 g du produit A préparé au stade A, 40 $cm^3$ de dioxane, 20 $cm^3$ d'eau et 500 mg d'acide paratoluène sulfonique. On maintient le mélange réactionnel au reflux sous agitation pendant 17 heures, le ramène à la température ambiante, dilue à l'eau et extrait au chlorure de méthylène. On lave les extraits réunis à l'eau. On sèche et distille à sec sous pression réduite, on obtient 3,2 g du produit recherché brut que l'on purifie par chromatographie sur silice en éluant par le mélange hexane-acétate d'éthyle (7-3). On obtient ainsi le produit recherché fondant à 61 °C $(\alpha)_D = +133°$ (c = 0,75 % DMF).

Exemple 11 : (2S,3S,3aS,5R,6aS) (hexahydro 3,5-méthano 2H-cyclopenta [b] furan-2-yl) oxy (S) α-cyano 3-phénoxy benzyle.

En opérant comme à l'exemple précédent à partir du lactol (2R) correspondant et de l'alcool (S) α-cyano 3-phénoxy benzylique, on a obtenu le produit recherché. F = 48 °C $(\alpha)_D = -121°$ (c = 0,5 % DMF).

Le lactol correspondant a été préparé comme suit :

A) (2S,3S,3aS,5R,6aS) (hexahydro 3,5-méthano 2H-cyclopenta [b] furan-2-yl) oxy (S) α-cyano 3-phénoxy benzyle.

On porte au reflux pendant 17 heures, 371 mg de (2R) (6-bromo hexahydro 3,5-méthano 2H-cyclopenta [b] furan-2-yl) oxy (S) α-cyano 3-phénoxy benzyle, 5 $cm^3$ de benzène d'hydrure de tributyl étain. On dilue à l'éther éthylique, agite avec une solution aqueuse de fluorure de potassium à 10 %, décante, lave à l'eau, sèche, distille sous pression réduite et obtient 380 mg d'un produit que l'on chromatographie sur silice en éluant par le mélange hexane-acétate d'éthyle (9-1). On obtient ainsi le produit recherché. F = 48 °C $(\alpha)_D = -121°$ (c = 0,5 % DMF).

B) Le (2R) hexahydro 3,5-méthano 2H-cyclopenta [b] furan-2-ol a été obtenu par hydrolyse du produit préparé au stade A.

Exemple 12 : (2R,3R,3aR,5S,6aR) (hexahydro 3,5-méthano 2H-cyclopenta [b] furan-2-yl) oxy (S) α-cyano 3-phénoxy benzyle.

En opérant comme à l'exemple 11 à partir du lactol et de l'alcool correspondant, on a obtenu le produit recherché. F < 45 °C $(\alpha)_D = +92,5°$ (c = 0,5 % DMF).

Préparation du lactol utilisé comme produit de départ :

Stade A : (2R,3R,3aR,5S,6aR) (hexahydro 3,5-méthano 2H-cyclopenta [b] furan-2-yl) oxy (S) α-cyano 3-phénoxybenzyle.

En opérant comme à l'exemple 11 à partir du dérivé bromé (2S) correspondant, on a obtenu le produit recherché. F < 45 °C $(\alpha)_D = +92,5°$ (c = 0,5 % DMF).

Stade B : (2S) hexahydro 3,5-méthano 2H-cyclopenta [b] furan-2-ol.

Le produit a été obtenu par hydrolyse du produit préparé au stade A.

Exemple 13 : (1R,2R,5S) 6,6-diméthyl 3-oxa 2-[(R) cyano-(6-phénoxy 2-pyridyl) méthoxy] bicyclo [3.1.0] hexane (produit A) et (1R,2R,5S) 6,6-diméthyl 3-oxa 2-[(S) cyano-(6-phénoxy 2-pyridyl) méthoxy] bicyclo [3.1.0] hexane (produit B).

Dans 50 $cm^3$ de chlorure de méthylène, on introduit 2,59 g de (1R,2S,5S) 6,6-diméthyl 3-oxa bicyclo hexane-2-ol, 4 g de cyano (6-phénoxy 2-pyridyl) méthanol racémique, 18 mg d'acide paratoluène sulfonique, agite à 20 °C pendant 17 heures, porte le mélange réactionnel au reflux sous pression réduite, en éliminant l'eau par distillation azéotropique, ajoute du bicarbonate de sodium, agite, concentre à sec par distillation sous pression réduite, chromatographie le résidu sur gel de silice en éluant par un

mélange d'éther de pétrole (Eb. 35-70 °C) et d'éther éthylique (8/2) et obtient 2,5 g de produit A $(\alpha)_D$ = + 95,5° (c = 0,9 % benzène) et 2,45 g de produit B $(\alpha)_D$ = + 69,6° (c = 0,9 % benzène).

Isomère A :

Spectre de RMN (deutérochloroforme)
— pic à 1,03 ppm attribué aux méthyl géminés du cyclopropyle.
— pics de 3,6 à 4,0 ppm attribués aux hydrogènes de $—OCH_2—$.
— pics à 5,2 et 5,4 ppm attribués à l'hydrogène de

$OCH<$

— pics à 6,8-6,9 ppm attribués aux hydrogènes en position 3 ou 5 du noyau pyridyle.
— pics à 7,6-7,7-7,8 ppm attribués à l'hydrogène en position 4 du noyau pyridyle.

Isomère B :

Spectre de RMN (deutérochloroforme)
— pics à 1,0-1,03 ppm attribués aux méthyles géminés du cyclopropyle,
— pics à 3,75-3,89 ppm et 4,12-4,25 ppm attribués à $—OCH_2—$
— pics à 5,0 et 5,3 ppm attribués aux hydrogènes de

$OCH<$

— pics à 6,75-6,9 ppm attribués aux hydrogènes en position 3 ou 5 du noyau pyridyle,
— pics à 7,6-7,7-7,8 ppm attribués à l'hydrogène en position 4 du noyau pyridyle.

Exemple 14 : (1R,2R,5S) (6,6-diméthyl 3-oxa 2-[(R) cyano-3-phénoxy 4-fluorophényl) méthoxy] bicyclo(3.1.0) hexane (produit A) et (1R,2R,5S) 6,6-diméthyl 3-oxa 2-[(S) cyano-3-phénoxy 4-fluorophényl) méthoxy] bicyclo(3.1.0) hexane (produit B).

On mélange 16 g d'alcool 4-fluoro 3-phénoxy (RS) α-cyano benzylique, 100 cm³ de dichlorométhane, 9,4 g de (1R,2S,5S), 6,6-diméthyl 3-oxa bicyclo(3.1.0) hexane 2-ol, 0,1 g d'acide paratoluène sulfonique, porte le mélange réactionnel au reflux, maintient le reflux pendant une heure et trente minutes, verse dans une solution aqueuse diluée de bicarbonate de potassium, sépare et concentre la phase organique à sec par distillation sous pression réduite et obtient 25,06 g de (1R,2R,5S) 6,6-diméthyl 3-oxa 2-[(RS) cyano-(3-phénoxy 4-fluorophényl) méthoxy] bicyclo(3.1.0.) hexane.

On chromatographie le produit obtenu sur silice en éluant par le mélange hexane-éther (8/2) et obtient 8,85 g du (1R,2R,5S) 6,6-diméthyl 3-oxa 2-[(R)-cyano (3-phénoxy 4-fluorophényl) méthoxy] bicyclo(3.1.0.) hexane. F < à 50 °C $(\alpha)_D$ = + 102° (c = 1 % benzène).

Les caractéristiques de ce produit sont les suivantes :

Dichroïsme circulaire (dioxane) :
— max à 279 nm Δε = —0,27.
Spectre de RMN (deutérochloroforme) :
— pic à 1,07 ppm attribué aux hydrogènes des méthyles géminés,
— pics à 1,33-1,78 ppm attribués aux hydrogènes du cyclopropyle,
— pics à 3,7-4,1 ppm attribués aux hydrogènes de $—CH_2O$.
— pics à 5,2-5,5 ppm attribués aux hydrogènes des

$O—CH<$

— pics à 6,9-7,6 ppm attribués aux hydrogènes des noyaux aromatiques.

En continuant la chromatographie, on obtient 9,05 g du (1R,2R,5S) 6,6-diméthyl 3-oxa 2-[(S) cyano-(3-phénoxy 4-fluorophényl) méthoxy] bicyclo(3.1.0) hexane, F = 65 °C. $(\alpha)_D$ = + 50° (c = 0,4 % benzène).

Les caractéristiques de ce produit sont les suivantes :

Dichroïsme circulaire (dioxane) :
— inflexion à 275 nm Δε = + 0,13 ; max à 281 nm Δε = + 0,15.
Spectre de RMN (deutérochloroforme) :
— pic à 1,0 ppm attribué aux hydrogènes des méthyles géminés.
— pics à 1,55-1,57 ppm attribués aux hydrogènes du cyclopropyle.

— pics à 3,8-3,9 ppm et 4,1-4,3 ppm attribués aux hydrogènes de $—CH_2O—$
— pics à 4,9-5,3 ppm attribués aux hydrogènes des

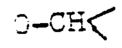

— pics à 6,9-7,6 ppm attribués aux hydrogènes des noyaux aromatiques.

En opérant comme précédemment à partir des lactols de formule III qui ont été décrits ci-dessus et des alcools de formule IV, on a obtenu les produits finals I, sous forme de diastéréoisomères que l'on a séparés, le cas échéant, en utilisant les procédés classiques de cristallisation et chromatographie.

Exemple 15 : (1R,2S,4R,5S) 6,6-diméthyl 4-trichlorométhyl 2-[1(S) 2-méthyl 4-oxo 3-(2-propen-1-yl) cyclopent-2-enyloxy] 3-oxo bicyclo(3.1.0) hexane.

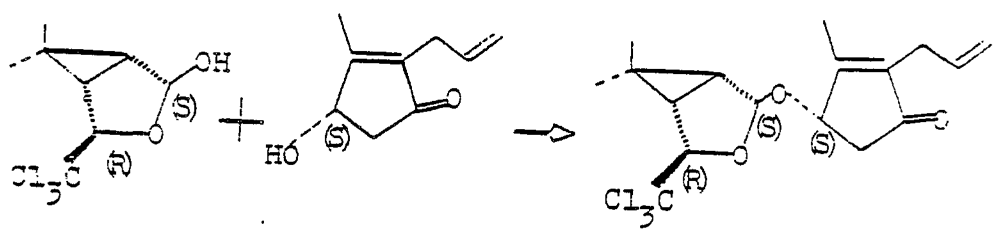

Exemple 16 : (1R,2S,4R,5S) 6,6-diméthyl 4-trichlorométhyl 2-[1(R) 2-méthyl 4-oxo 3-(2-propen-1-yl) cyclopent-2-enyloxy] 3-oxo bicyclo(3.1.0) hexane.

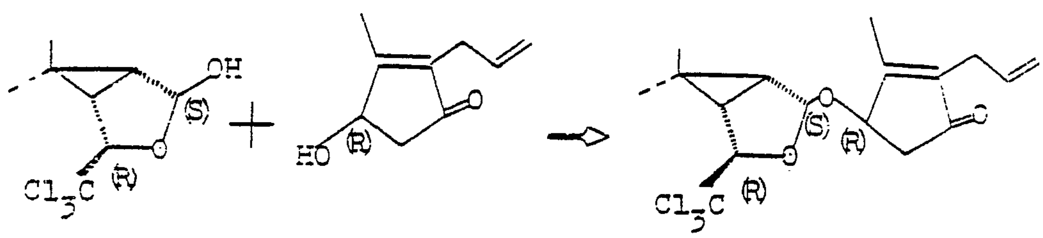

Exemple 17 : (1R,2S,4R,5S) 6,6-diméthyl 4-trichlorométhyl 2-[RS cyano(3-phénoxyphényl) méthoxy] 3-oxo bicyclo(3.1.0) hexane et isomères R et S séparés.

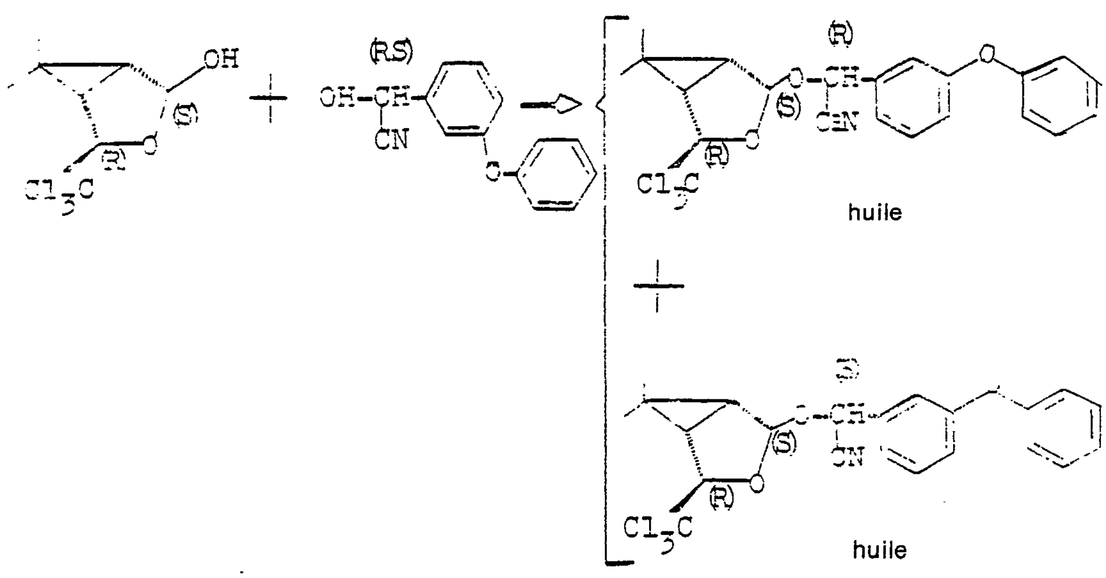

Exemple 18 : (1R,2S,4S,5S) 6,6-diméthyl 4-tribromométhyl 2-[1(S) 2-méthyl 4-oxo 3-(2-propen-1-yl) cyclopent-2-enyloxy] 3-oxa bicyclo(3.1.0) hexane.

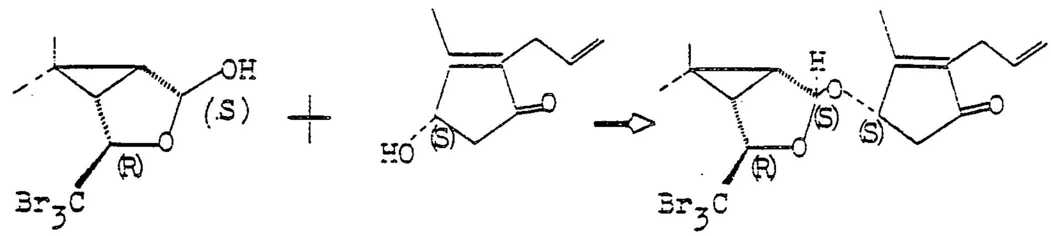

huile

(α)$_D$ = + 72° ± 3

(c = 0,6 % benzène)

Exemple 19 : (11R,12S,15S) 12-[S(1-méthyl) heptyloxy] 9,10,11,12,14,15-hexahydro 9,10 [3',4'] furano anthracène et (11S,12R,15R) 12-[S(1-méthyl) heptyloxy] 9,10,11,12,14,15-hexahydro 9,10 [3',4'] furano anthracène.

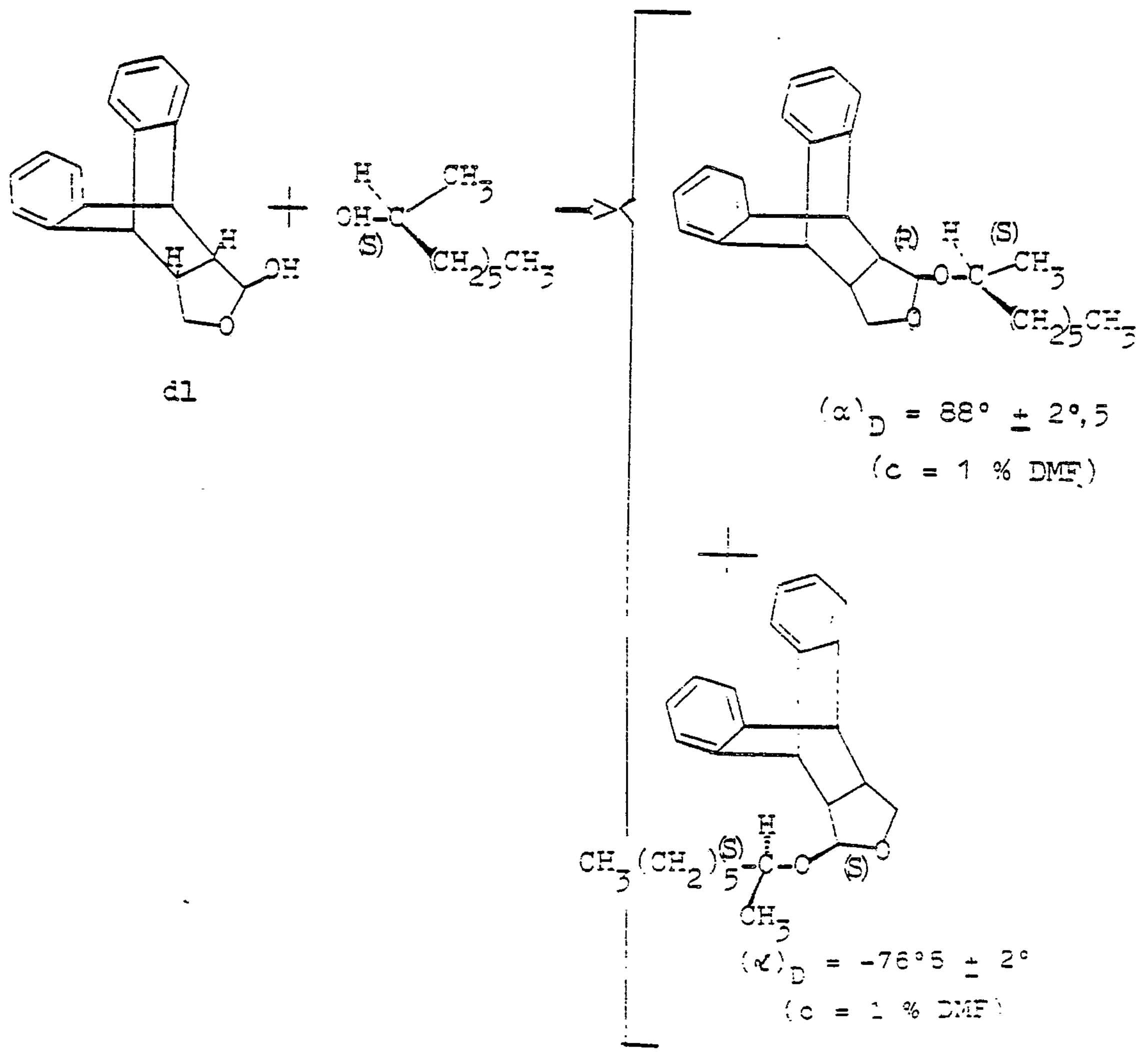

Exemple 20 : (11R,12S,15S) 12-[1S 2-méthyl 4-oxo 3-(2-propen-1-yl) cyclopent-2-enyloxy] 9,10,11,12,14,15-hexahydro 9,10 [3',4'] furano anthracène et (11R,12S,15S) 12-[1R 2-méthyl 4-oxo 3-(2-propen-1-yl) cyclopent-2-enyloxy] 9,10,11,12,14,15-hexahydro 9,10 [3',4'] furano anthracène.

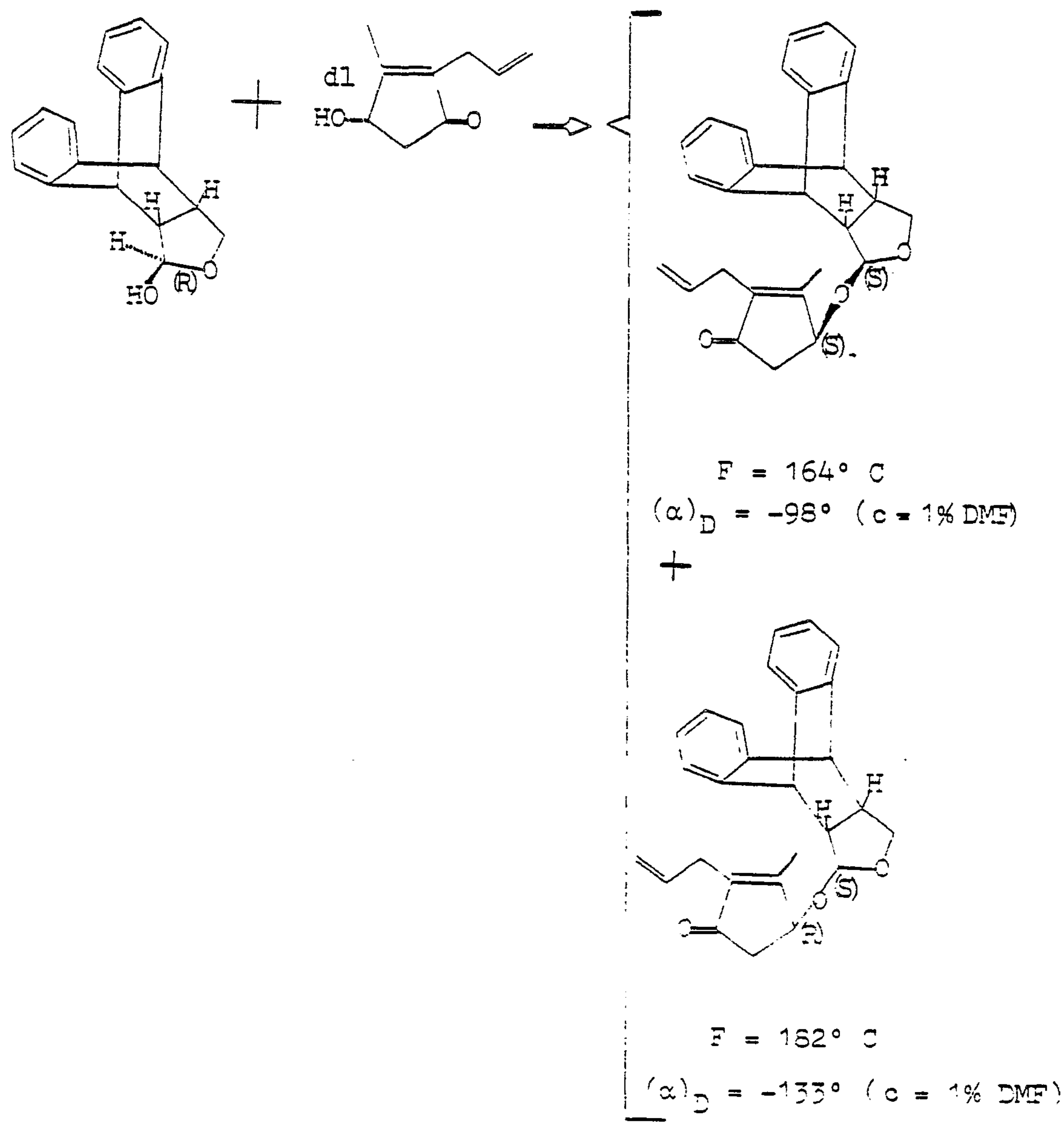

Exemple 21 : (11S,12R,15R) 12-[(R) phényl éthyloxy] 9,10,11,12,14,15-hexahydro 9,10 [3',4'] furano anthracène et (11S,12R,15R) 12-[(S) 1-phényl éthyloxy] 9,10,11,12,14,15-hexahydro 9,10 [3',4'] furano anthracène.

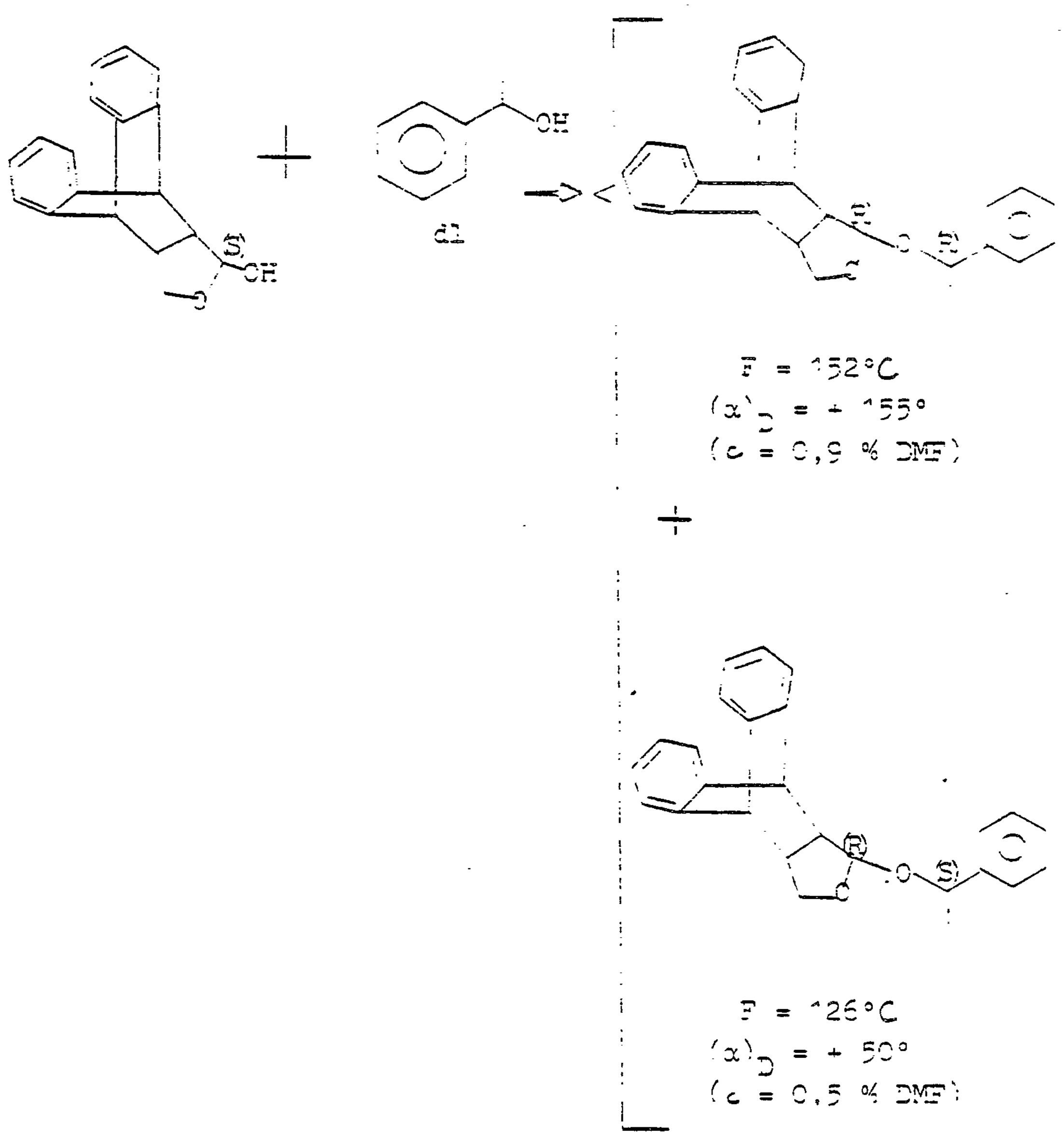

Exemple 22 : (11R,12S,15S) 12-[(R) 3-pinanyl méthoxy] 9,10,11,12,14,15-hexahydro 9,10 [3',4'] furano anthracène et (11R,12S,15S) 12-[(S) 3-pinanyl méthoxy] 9,10,11,12,14,15-hexahydro 9,10 [3',4'] furano anthracène.

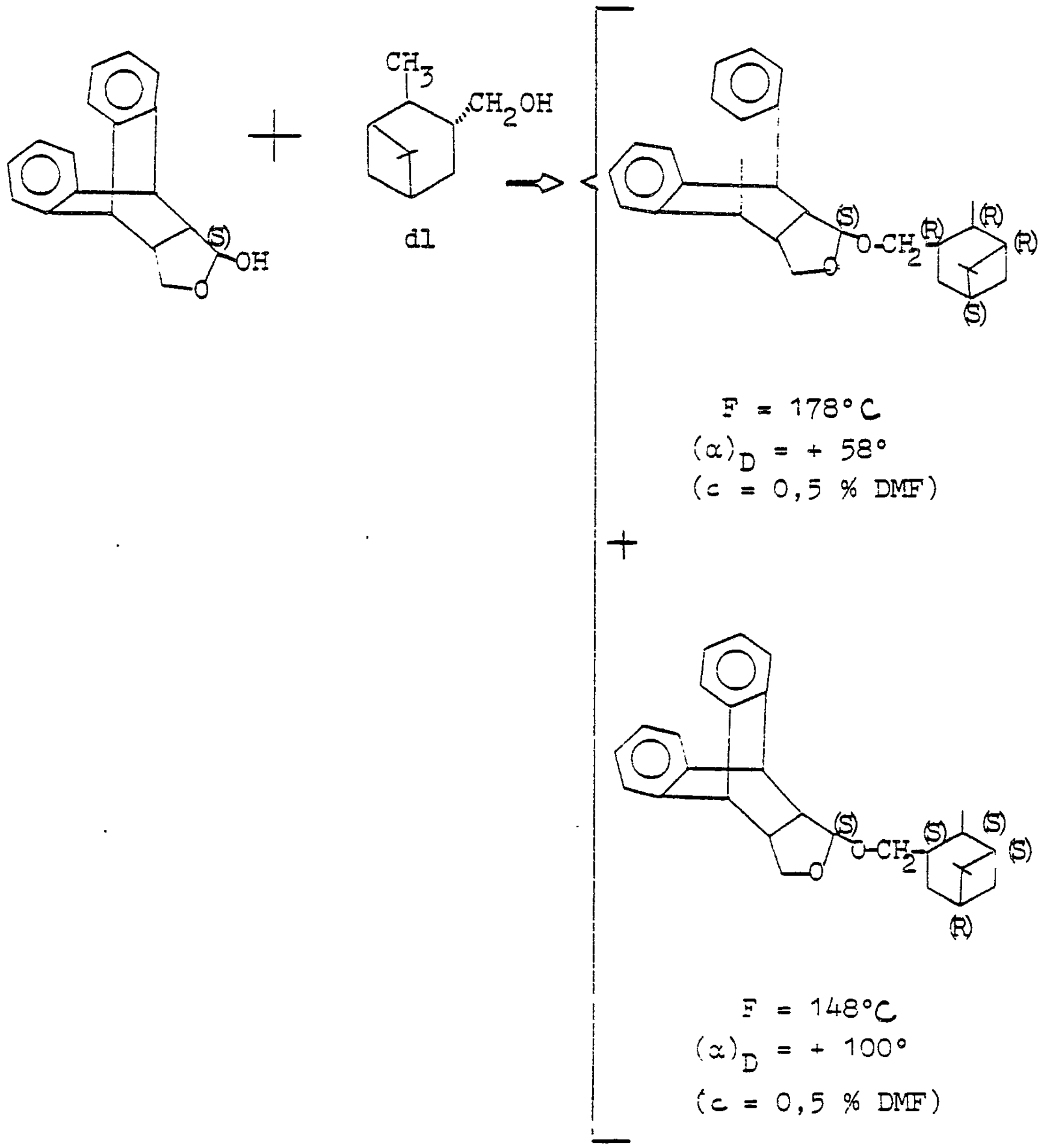

Exemple 23 : (11R,12S,15S) 12-[(S) 2-méthyl butyloxy] 9,10,11,12,14,15-hexahydro 9,10 [3',4'] furano anthracène et (11R,12S,15S) 12-[(R) 2-méthyl butyloxy] 9,10,11,12,14,15-hexahydro 9,10 [3',4'] furano anthracène.

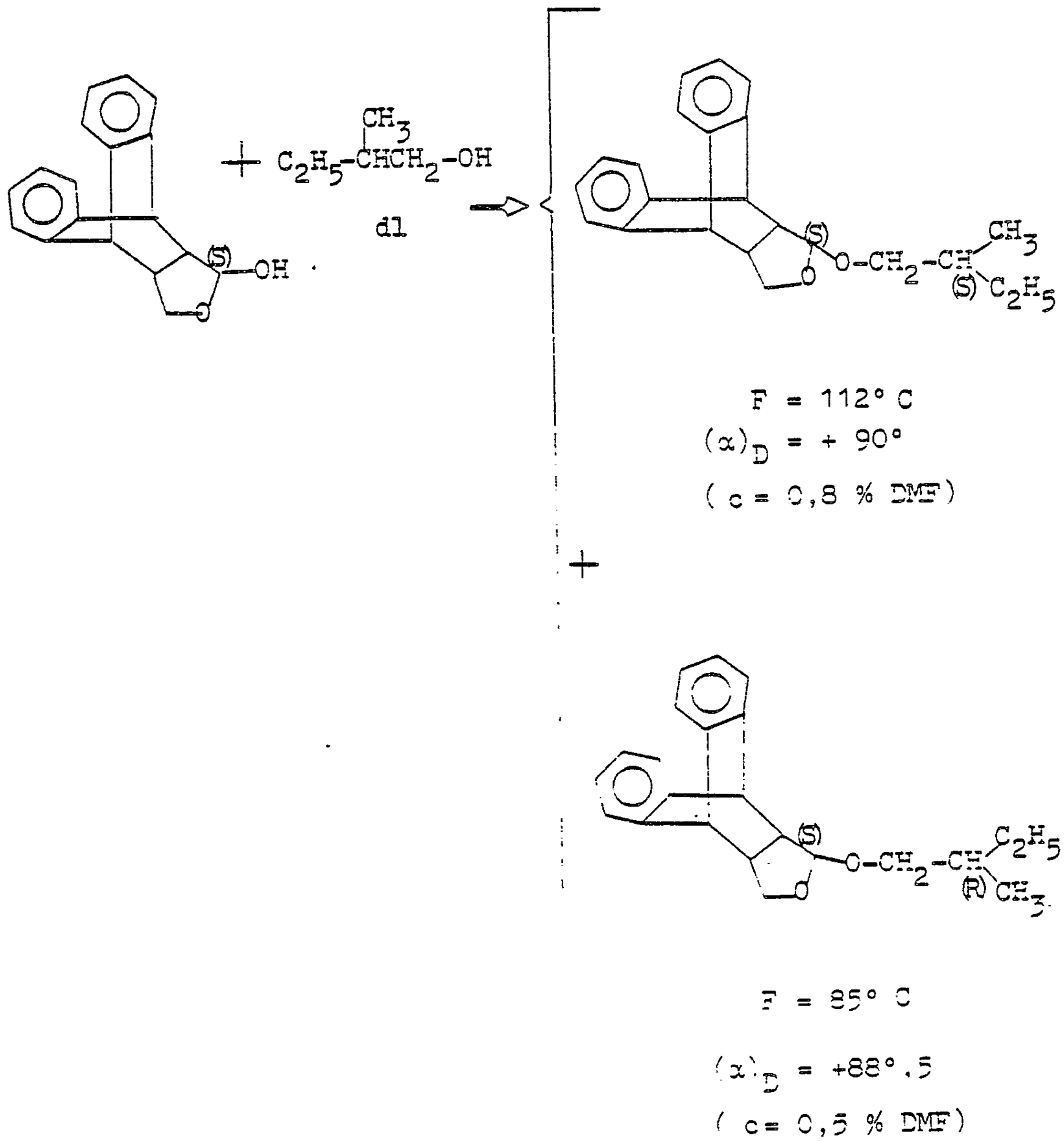

Exemple 24 : (11S,12R,15R) 12-[(S) 1-méthyl butyloxy] 9,10,11,12,14,15-hexahydro 9,10 [3',4'] furano anthracène et (11S,12R,15R) 12-[(R) 1-méthyl butyloxy] 9,10,11,12,14,15-hexahydro 9,10 [3',4'] furano anthracène.

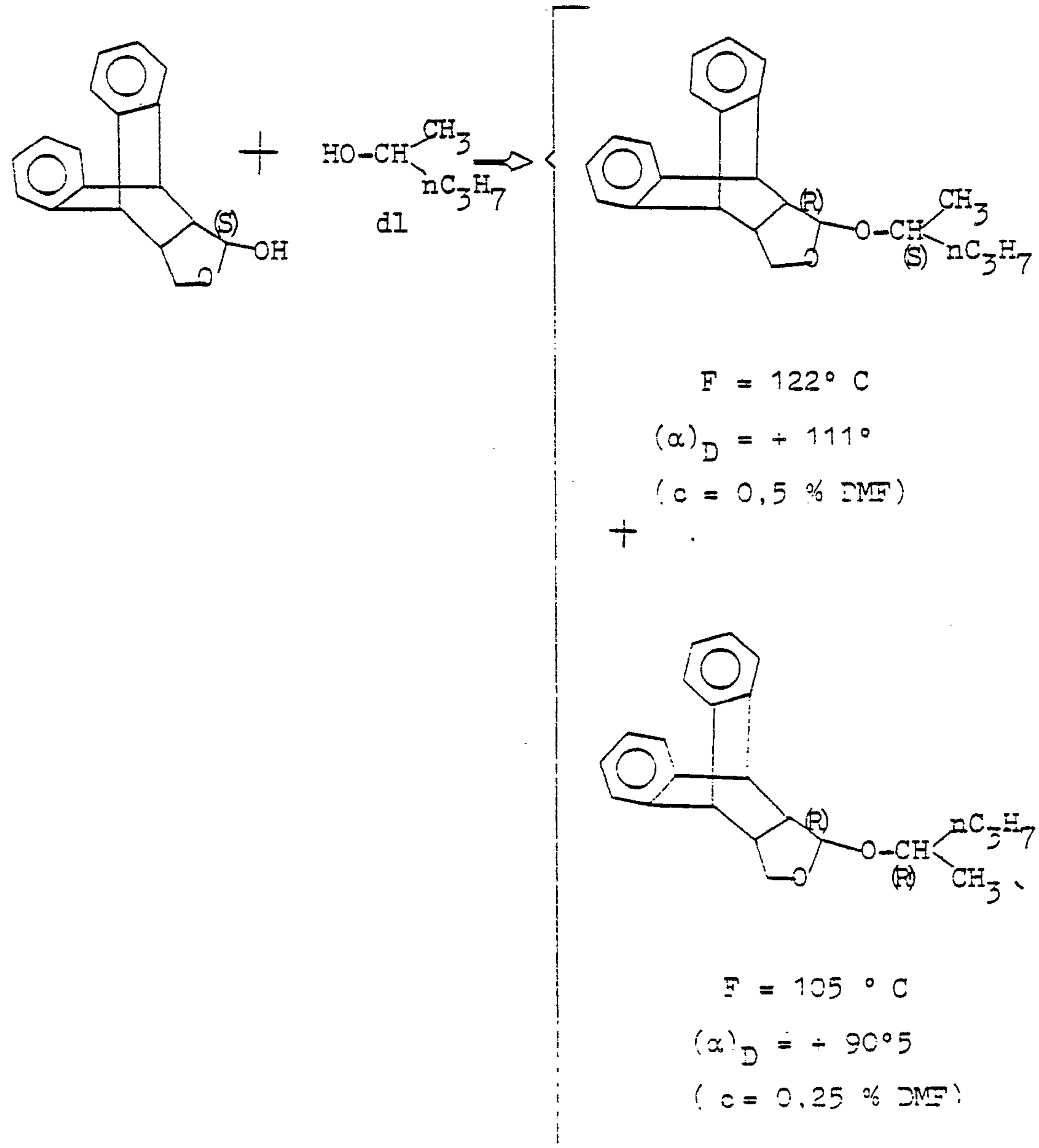


Les exemples d'application suivants montrent quelques unes des possibilités d'utilisation des produits de formule I pour préparer soit des produits de formule III optiquement actifs

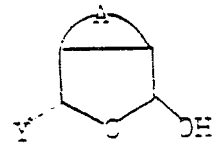

(III)

A et Y conservant leur signification précédente, soit des alcools de formule IV optiquement actifs

ROH (IV)

R conservant sa signification précédente.

Application :

Exemple 25 : Alcool (S) α-cyano 3-phénoxy benzylique.

On mélange 1 g de (1R,2R,5S) 6,6-diméthyl 3-oxa 2-[S-cyano (3-phénoxyphényl) méthoxy] bicyclo(3.1.0) hexane obtenu à l'exemple 1, 10 $cm^3$ de méthanol et 0,1 g d'acide paratoluène sulfonique, agite pendant 2 heures à 20 °C, ajoute de l'eau, extrait au chlorure de méthylène, concentre à sec par distillation sous pression réduite, chromatographie le résidu sur silice en éluant avec un mélange d'hexane et d'acétate d'éthyle (7/3) et obtient le produit recherché.
$(\alpha)_D$ = — 16°5 (c = 0,8 % benzène).

Alcool (R) α-cyano 3-phenoxy benzylique

On mélange 1 g de (1R,2R,5S) 6,6-diméthyl 3-oxa 2-[R cyano-(3-phénoxyphényl) méthoxy] bicyclo(3.1.0) hexane obtenu à l'exemple 1, 10 $cm^3$ de méthanol et 0,1 g d'acide paratoluène sulfonique, agite pendant 2 heures à 20 °C, ajoute de l'eau, extrait au chlorure de méthylène, concentre à sec par distillation sous pression réduite, chromatographie le résidu sur silice en éluant avec un mélange d'hexane et d'acétate d'éthyle (7/3) et obtient le produit recherché.
$(\alpha_D)$ = + 16°5 (c = 0,8 % benzène).

Application :

Exemple 26 : Alcool 4-fluoro 3-phénoxy (R) α-cyano benzylique.

On mélange 9 g de (1R,2R,5S) 6,6-diméthyl 3-oxa 2-[(R) cyano (3-phénoxy 4-fluorophényl) méthoxy] bicyclo(3.1.0) hexane obtenu à l'exemple 14, 100 $cm^3$ de méthanol, 90 mg d'acide paratoluène sulfonique, agite à 20 °C, pendant 1 heure et trente minutes, verse le mélange dans l'eau, extrait au chloroforme, concentre la phase organique à sec par distillation sous pression réduite, chromatographie le résidu sur silice en éluant par un mélange d'hexane et d'acétate d'éthyle (7/3) à 1 % d'acide acétique.

On obtient 4,9 g d'alcool 4-fluoro 3-phénoxy (R) α-cyano benzylique. $(\alpha)_D$ = + 26°5 ± 2°5 (c = 0,5 % pyridine).

Alcool 4-fluoro 3-phénoxy (S) α-cyano benzylique

En opérant comme indiqué dans l'exemple de préparation de l'alcool 4-fluoro 3-phénoxy (R) α-cyano benzylique, à partir de 9 g de (1R,2R,5S) 6,6-diméthyl 3-oxa 2-[(S) cyano-(3-phénoxy 4-fluorophényl) méthoxy] bicyclo(3.1.0) hexane obtenu à l'exemple 14, on obtient 4,5 g d'alcool 4-fluoro 3-phénoxy (S) α-cyano benzylique.
$(\alpha_D)$ = — 30° ± 2°5 (c = 0,5 % pyridine).

Application :

Exemple 27 : Alcool (R) cyano(6-phénoxy 2-pyridyl) méthylique.

Dans 200 $cm^3$ de méthane, on introduit 13,4 g de (1R,2R,5S) 6,6-diméthyl 3-oxo 2-[(R) cyano(6-phénoxy 2-pyridyl) méthoxy] bicyclo(3.1.0) hexane (isomère A), obtenu à l'exemple 13, 150 mg d'acide paratoluène sulfonique, agite pendant 1 heure et 30 minutes à 20 °C, verse dans un mélange d'eau et de glace, isole par essorage le précipité formé et obtient 8 g de produit recherché.
F = 95 °C.

Exemple 28 : Alcool (S) cyano(6-phénoxy 2-pyridyl) méthylique.

En opérant de façon analogue à celle de l'exemple 27, au départ de 13,4 g de (1R,2R,5S) 6,6-diméthyl 3-oxa 2-[(S) cyano(6-phénoxy 2-pyridyl) méthoxy] bicyclo(3.1.0) hexane (isomère B), décrit dans l'exemple 13, on obtient 8 g de produit recherché.
F = 95 °C $(\alpha)_D$ = + 62 °5 (c = 1 % pyridine)
$(\alpha)_D$ = — 80° (c = 1 % benzène).

De la même façon que précédemment à partir des produits de formule I correspondants, on a obtenu par hydrolyse, les produits suivants :

Exemple 29 : R-alléthrolone et (1R,2S,4R,5S) 6,6-diméthyl 4-(trichlorométhyl) 3-oxa bicyclo(3.1.0) hexan-2-ol.

Voir schéma p. 25

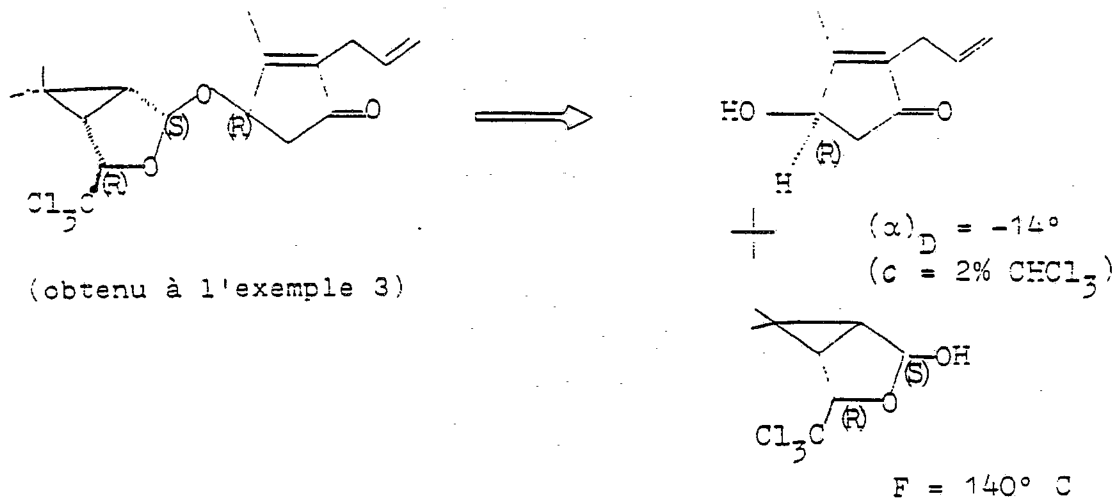


Exemple 30 : R-alléthrolone et (1R,2S,4R,5S) 6,6-diméthyl 4-tribromométhyl 3-oxa bicyclo(3.1.0) hexan-2-ol.

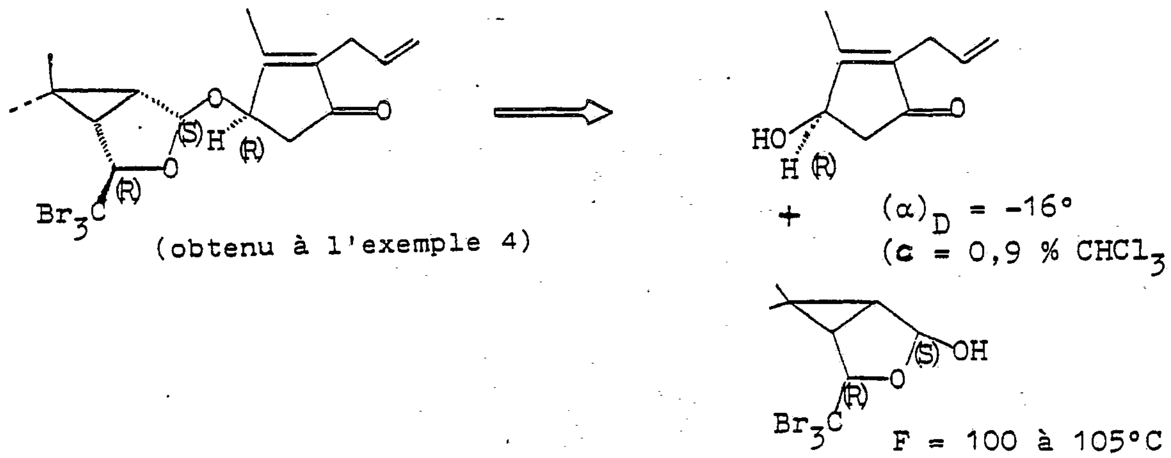


Exemple 31 : (11R,12R,15S) 9,10,11,12,14,15-hexahydro 9,10 [3',4'] furano anthracène-12-ol et alcool (S) α-cyano 3-phénoxy benzylique.

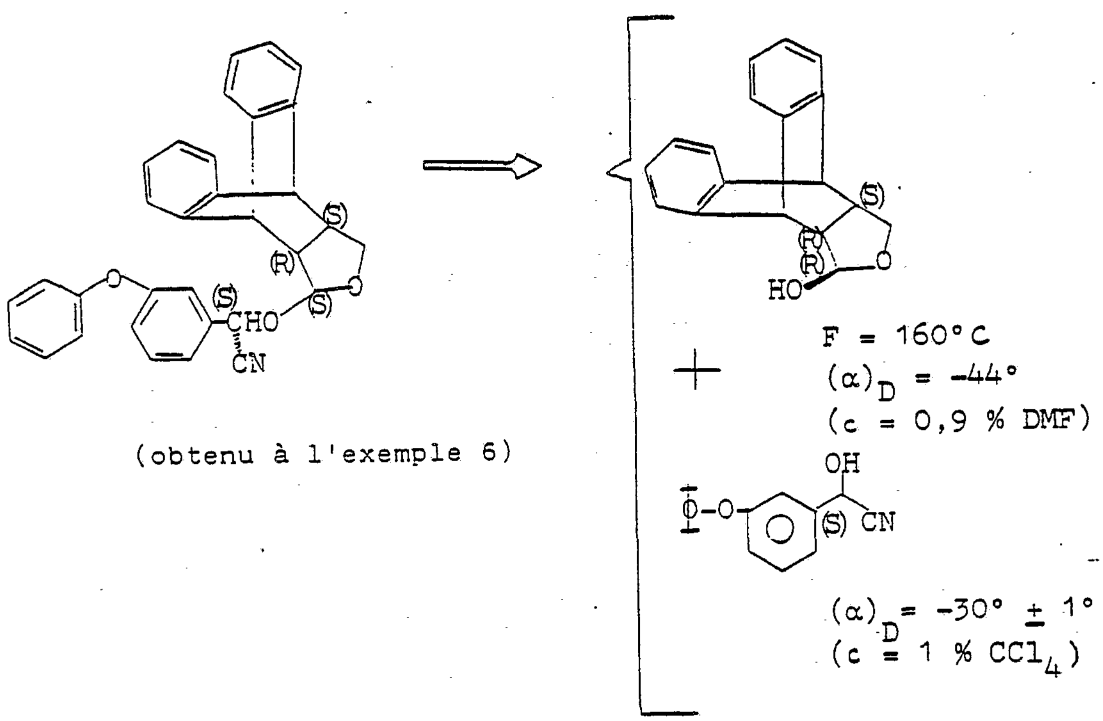

Exemple 32 : (11R,12R,15S) 9,10,11,12,14,15-hexahydro 9,10 [3',4'] furano anthracène 12-ol et 2(S) octanol.

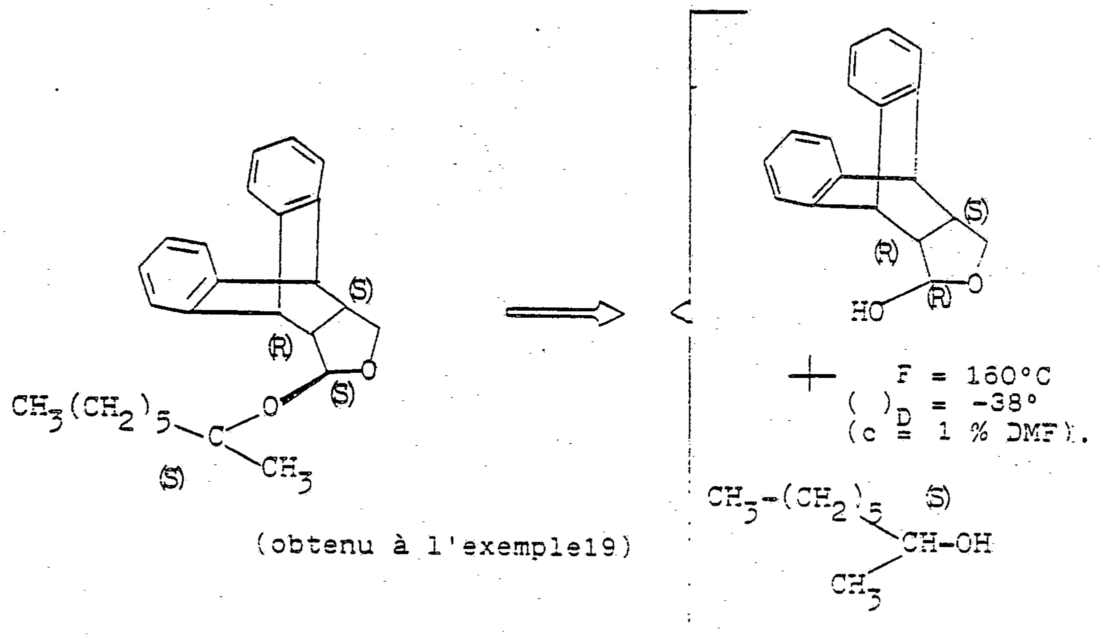


Exemple 33 : Alcool (R) α-cyano 3-phénoxy benzylique et (2S) 6-bromo hexahydro 3,5-méthano 2H-cyclopenta[b]furan-2-ol.

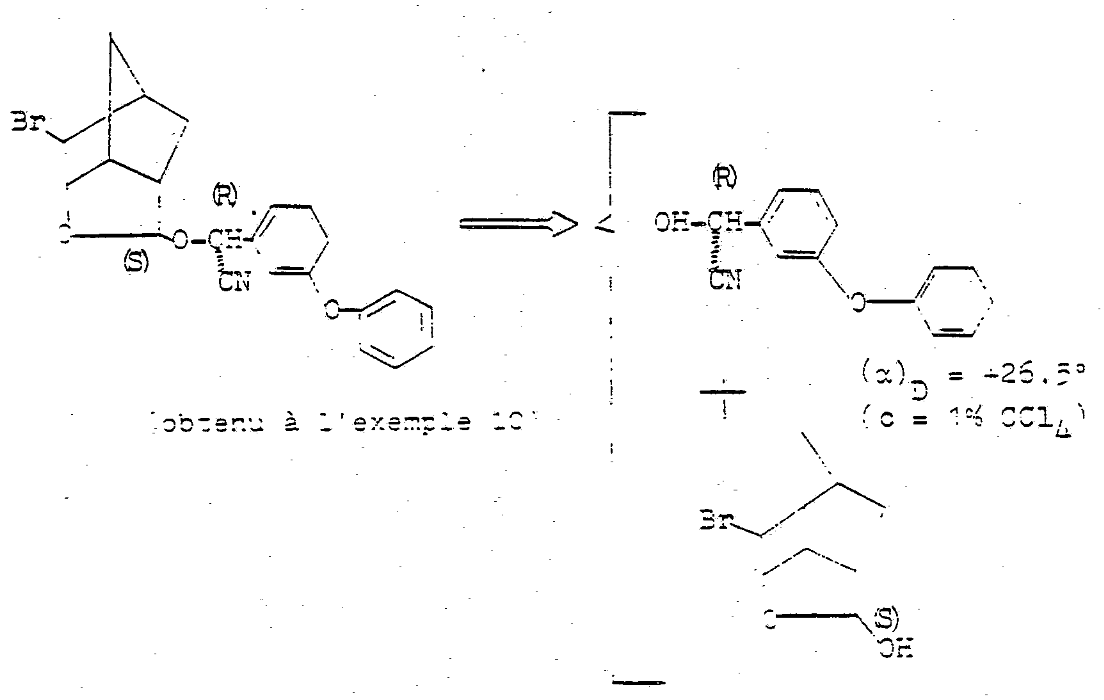

Exemple 34 : Alcool (S) α-cyano 3-phénoxybenzylique et (2S) 6-bromohexahydro 3,5-méthano 2H-cyclopenta [b] furan-2-ol.

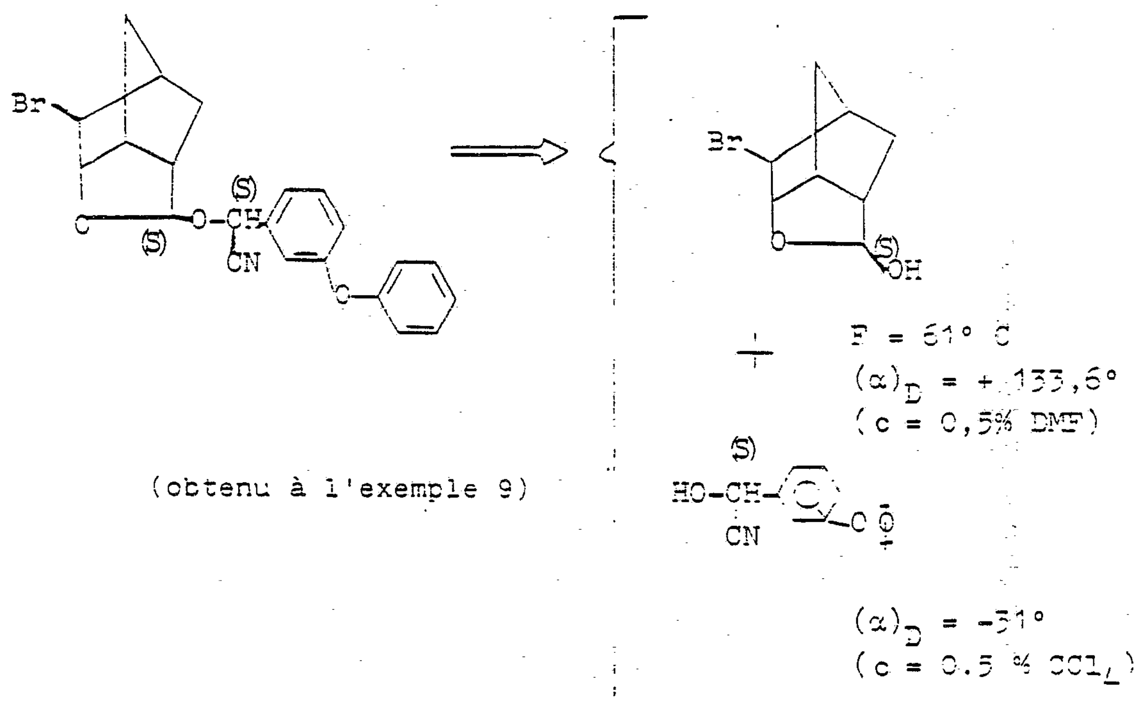


Exemple 35 : (2R) 6-bromo hexahydro 3,5-méthano 2H-cyclopenta [b] furan-2-ol et alcool (S) α-cyano 3-phénoxy benzylique.

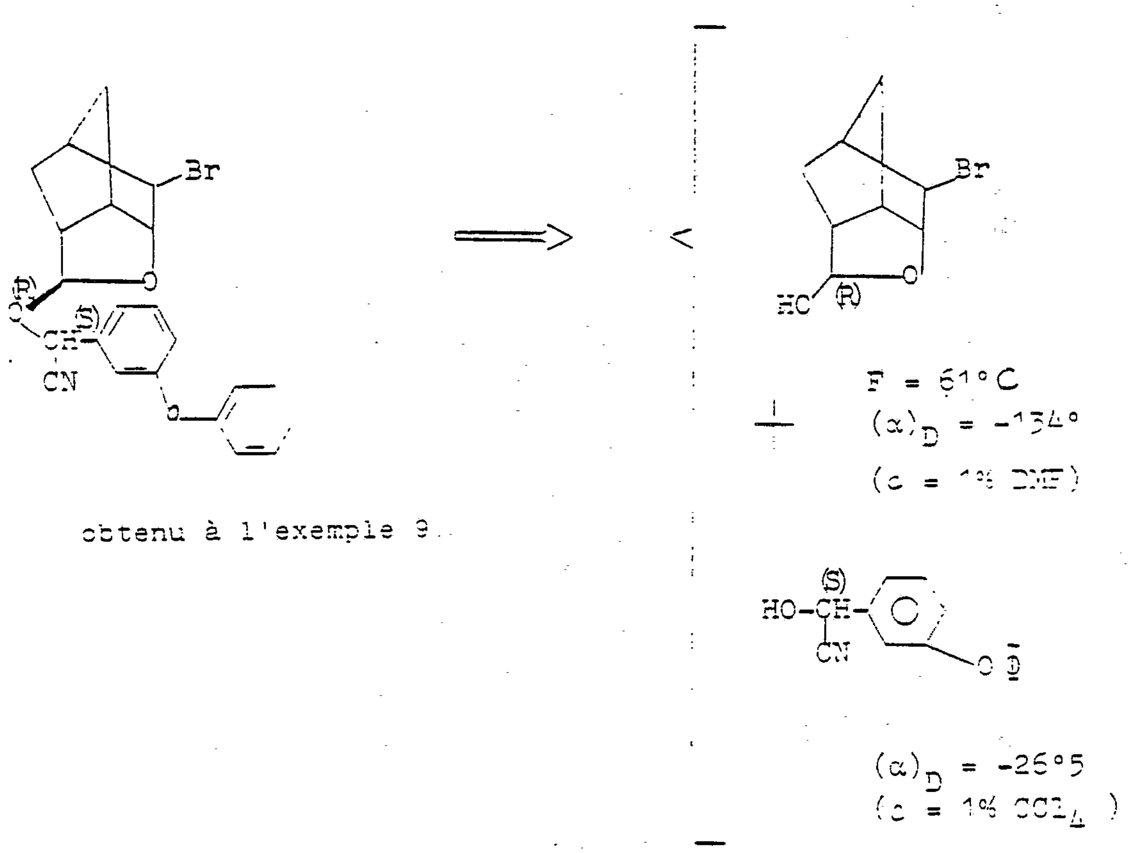

**Revendications** (pour les Etats contractants : BE, CH, DE, FR, GB, IT, LI, LU, NL, SE)

1. Sous toutes les formes isomères possibles, les composés de formule générale (I) :

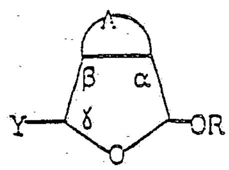

(I)

formule dans laquelle le symbole A représente une chaîne hydrocarbonée comportant de 1 à 16 chaînons, cette chaîne pouvant comporter un ou plusieurs hétéroatomes, une ou plusieurs insaturations, l'ensemble des chaînons constitutifs de la chaîne pouvant représenter un système mono ou polycyclique, y compris un système du type spiro ou endo, l'ensemble constitué par la chaîne A et les atomes de carbone auxquels elle est liée, pouvant comporter un ou plusieurs atomes chiraux ou bien la copule hémiacétalique pouvant présenter une chiralité due à la configuration spatiale dissymétrique de l'ensemble de la molécule, R représente soit un reste d'alcool primaire, secondaire ou tertiaire, comportant au moins un carbone asymétrique, soit un reste alcoolique substitué dont la chiralité est due à la configuration spatiale dissymétrique de l'ensemble de la molécule, Y représente soit un atome d'hydrogène, soit un groupement $-CY'_3$ dans lequel Y' est un atome de chlore ou de brome, soit un groupement alcoyle, linéaire ou ramifié, comportant de 1 à 18 atomes de carbone, éventuellement substitué, la liaison βγ de la formule I ainsi que le substituant Y pouvant dans ce dernier cas, faire partie du substituant A, ainsi que les mélanges de ces isomères.

2. Composés selon la formule I de la revendication 1, dans lesquels les atomes ou radicaux différents qui substituent le ou les atomes de carbone portés par la chaîne A ou situés en α ou β, sont choisis indifféremment dans l'un ou l'autre des groupes suivants :

a) le groupe constitué par l'hydrogène, les atomes d'halogènes, le groupement nitro, les radicaux alcoyles comportant de 1 à 18 atomes de carbone, les radicaux cyclo alcoyles comportant de 3 à 6 atomes de carbone, le radical phényle, les radicaux phényles substitués soit par un ou plusieurs atomes d'halogènes, soit par un ou plusieurs radicaux alcoyles comportant de 1 à 6 atomes de carbone,

b) le groupe constitué par les radicaux :

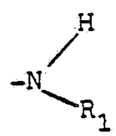

dans lesquels $R_1$ représente un atome d'hydrogène, un radical alcoyle comportant de 1 à 6 atomes de carbone, le radical :

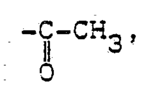

le radical :

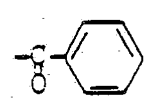

ou le radical :

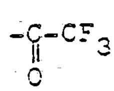

c) le groupe constitué par les radicaux :

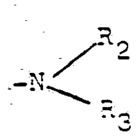

dans lesquels $R_2$ et $R_3$, identiques ou différents, représentent un radical alcoyle comportant de 1 à 6 atomes de carbone ou bien dans lesquels $R_2$ et $R_3$ représentent ensemble avec l'atome d'azote auquel ils sont liés, un hétérocycle comportant 6 atomes.

3. Composés selon la revendication 1 ou 2, caractérisés en ce que la chaîne A a pour structure :

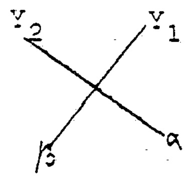

$Y_1$ et $Y_2$, identiques ou différents, représentant un atome d'hydrogène, de fluor, de chlore ou de brome ou un radical alcoyle comportant de 1 à 6 atomes de carbone ou $Y_1$ et $Y_2$ formant ensemble avec l'atome de carbone auquel ils sont liés, un homocycle carboné comportant de 3 à 7 atomes de carbone.

4. Composés selon la revendication 1, 2 ou 3, caractérisés en ce que la chaîne A a pour structure :

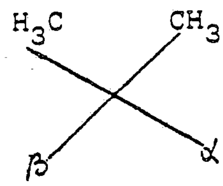

5. Composés selon la revendication 1 ou 2, caractérisés en ce que la chaîne A a pour structure :

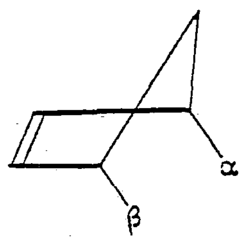

6. Composés selon la revendication 1 ou 2, caractérisés en ce que la chaîne A a pour structure :

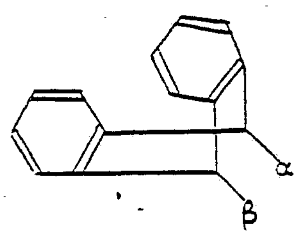

7. Composés selon la revendication 1 ou 2, caractérisés en ce que la chaîne A a pour structure :

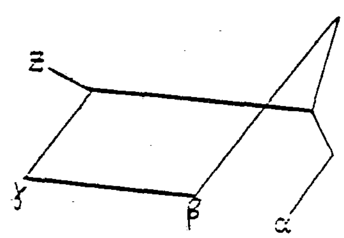

formule dans laquelle Z représente un atome d'hydrogène, un atome de chlore, un atome de brome ou d'iode.

8. Composés selon l'une quelconque des revendications 1 à 7, caractérisés en ce que le substituant R représente un reste cyanométhyle substitué, choisi dans le groupe constitué par les radicaux :

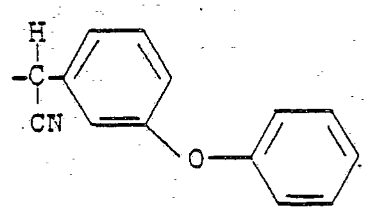

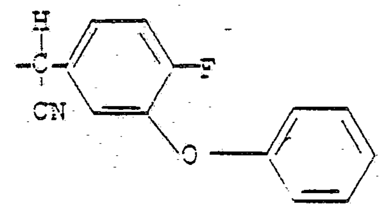

et

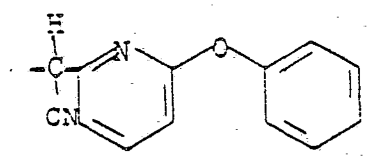

,

ainsi que par les radicaux correspondants, dans lesquels le groupement cyano est remplacé par un radical alkyle, alkényle ou alkynyle.

9. Composés selon l'une quelconque des revendications 1 à 7, caractérisés en ce que le substituant R a pour formule :

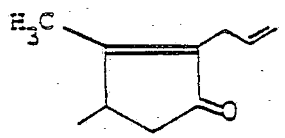

10. L'un quelconque des composés de formule générale (I), selon la revendication 1, dont les noms suivent :

le (1R,2R,4R,5S) 6,6-diméthyl 3-oxa 2-[(S) cyano(3-phénoxyphényl) méthoxy] 4-(2-méthyl 2-propyl) bicyclo[3.1.0] hexane ;

le (1R,2R,4R,5S) 6,6-diméthyl 3-oxa 2-[(R) cyano(3-phénoxyphényl) méthoxy] 4-(2-méthyl 2-propyl) bicyclo[3.1.0] hexane ;

le (1R,2S,4R,5S) 6,6-diméthyl 4-trichlorométhyl 2-[(R) 2-méthyl 4-oxo 3-(2-propen-1-yl) cyclopent-2-enyloxy] 3-oxa bicyclo(3.1.0) hexane ;

le (1R,2S,4R,5S) 6,6-diméthyl 4-trichlorométhyl 2-[(S) 2-méthyl 4-oxo 3-(2-propen-1-yl) cyclopent-2-enyloxy] 3-oxa bicyclo(3.1.0) hexane ;

le (1R,2R,5S) 6,6-diméthyl 3-oxa 2-[(S) cyano(3-phénoxyphényl) méthoxy] bicyclo(3.1.0) hexane ;

le (1R,2R,5S) 6,6-diméthyl 3-oxa 2-[(R) cyano(3-phénoxyphényl) méthoxy] bicyclo(3.1.0) hexane ;

le (1R,2S,4R,5S) 6,6-diméthyl 4-trichlorométhyl 2-[(S) cyano(3-phénoxyphényl) méthoxy] 3-oxa bicyclo(3.1.0) hexane ;

le (1R,2S,4R,5S) 6,6-diméthyl 4-trichlorométhyl 2-[(R) cyano(3-phénoxyphényl) méthoxy] 3-oxa bicyclo(3.1.0) hexane ;

le (1R,2S,4R,5S) 6,6-diméthyl 4-tribromométhyl 2-[(R) 2-méthyl 4-oxo 3-(2-propen-1-yl) cyclopent-2-enyloxy] 3-oxa bicyclo(3.1.0) hexane ;

le (1R,2S,4R,5S) 6,6-diméthyl 4-tribromométhyl 2-[(S) 2-méthyl 4-oxo 3-(2-propen-1-yl) cyclopent-2-enyloxy] 3-oxa bicyclo(3.1.0) hexane ;

le (1R,2R,5S) 6,6-diméthyl 3-oxa 2-[(R) cyano(3-phénoxy 4-fluoro phényl) méthoxy] bicyclo(3.1.0) hexane ;

le (1R,2R,5S) 6,6-diméthyl 3-oxa 2-[(S) cyano(3-phénoxy 4-fluoro phényl) méthoxy] bicyclo(3.1.0) hexane ;

le (1R,2R,5S) 6,6-diméthyl 3-oxa 2-[(S) cyano(6-phénoxy 2-pyridinyl) méthoxy] bicyclo(3.1.0) hexane ;

le (1R,2R,5S) 6,6-diméthyl 3-oxa 2-[(R) cyano(6-phénoxy 2-pyridinyl) méthoxy] bicyclo(3.1.0) hexane.

11. L'un quelconque des composés de formule générale (I), selon la revendication 1, dont les noms suivent :

le (3aR,4R,7S,7aS,1R) 1-[(R) cyano(3-phénoxyphényl) méthoxy] tétrahydro 4,7-méthano isobenzofurane ;

le (3aR,4R,7S,7aS,12) 1-[(S) cyano(3-phénoxyphényl) méthoxy] tétrahydro 4,7-méthano isobenzofurane.

12. L'un quelconque des composés de formule générale (I), selon la revendication 1, dont les noms suivent :

le (11S,12R,15R) 12-[(S) cyano(3-phénoxyphényl) méthoxy] 9,10,11,12,14,15-hexahydro 9,10 [3',4'] furano anthracène ;

le (11R,12S,15S) 12-[(S) cyano(3-phénoxyphényl) méthoxy] 9,10,11,12,14,15-hexahydro 9,10 [3',4'] furano anthracène ;

le (11R,12S,15S) 12-[(1S) 2-méthyl 4-oxo 3-(2-propen-1-yl) cyclopent-2-enyloxy] 9,10,11,12,14,15-hexahydro 9,10 [3',4'] furano anthracène ;

le (11R,12S,15S) 12-[(1R) 2-méthyl 4-oxo 3-(2-propen-1-yl) cyclopent-2-enyloxy] 9,10,11,12,14,15-hexahydro 9,10 [3',4'] furano anthracène ;

le (11R,12S,15S) 12-[(R) (4,4-diméthyl 2-oxo tétrahydrofuran-3-yl) oxy] 9,10,11,12,14,15-hexahydro 9,10 [3',4'] furano anthracène ;

le (11R,12S,15S) 12-[(S) (4,4-diméthyl 2-oxo tétrahydrofuran-3-yl) oxy] 9,10,11,12,14,15-hexahydro 9,10 [3',4'] furano anthracène ;

le (11S,12R,15S) 12-[(1R,2S,5R) 2-prop-2-yl 5-méthyl cyclohexyloxy] 9,10,11,12,14,15-hexahydro 9,10 [3',4'] furano anthracène ;

le (11R,12S,15S) 12-[(1R,2S,5R) 2-prop-2-yl 5-méthyl cyclohexyloxy] 9,10,11,12,14,15-hexahydro 9,10 [3',4'] furano anthracène ;

13. L'un quelconque des composés de formule générale (I), selon la revendication 1, dont les noms suivent :

le [(12R) (6-bromohexahydro 3,5-méthano 2H-cyclopenta [b] furan-2-yl) oxy] (S) α-cyano 3-phénoxy benzyle,

le [(2S) (6-bromohexahydro 3,5-méthano 2H-cyclopenta [b] furan-2-yl) oxy] (S) α-cyano 3-phénoxy benzyle.

14. Procédé de préparation des composés de formule générale (I) tels que définis à la revendication 1, caractérisé en ce que l'on fait réagir l'hydrure de diisobutyl aluminium au sein d'un solvant organique sur un composé racémique ou optiquement actif de formule générale II :

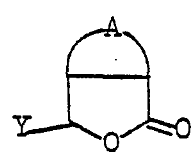

(II)

dans laquelle A et Y conservent les significations précitées, puis soumet le composé de formule générale III résultant :

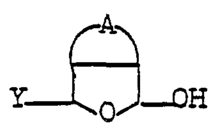

(III)

à l'action d'un alcool racémique ou optiquement actif de formule générale IV :

$$R—OH \quad (IV)$$

formule dans laquelle R conserve les significations précitées pour obtenir le composé de formule I correspondant, dont on sépare, le cas échéant, les diastéréoisomères.

15. Procédé selon la revendication 14, utilisable lorsque Y représente un atome d'hydrogène, caractérisé en ce que l'on fait réagir un hydroborure alcalin au sein d'un solvant, puis un acide au sein d'un solvant organique, avec un composé de formule générale V :

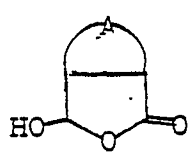

(V)

dans laquelle A conserve la signification précédente, pour obtenir le composé de formule générale $II_A$ :

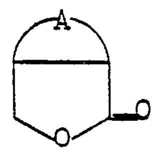

($II_A$)

que l'on traite selon le procédé de la revendication 11.

16. Application des composés de formule générale I tels que définis à la revendication 1 au dédoublement des composés de formule générale III ou IV, application qui consiste à faire réagir : ou bien un composé hémiacétalique optiquement actif de formule générale III :

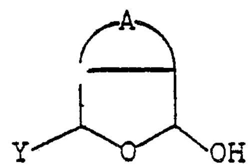

(III)

dans laquelle Y et A conservent leurs significations précitées, avec un alcool racémique de formule générale IV :

R—OH (IV)

dans laquelle R conserve sa signification précédente,
ou bien un composé hémiacétalique racémique de formule générale III :

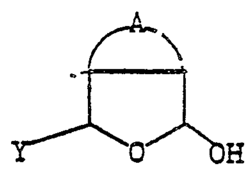

(III)

dans laquelle Y et A conservent leur signification précédente avec un alcool optiquement actif de formule générale IV :

R—OH (IV)

dans laquelle R conserve sa signification précédente, pour obtenir un mélange de diastéréoisomères de formule I que l'on sépare selon les procédés classiques, application caractérisée en ce que l'on clive lesdits diastéréoisomères de formule I, pour obtenir respectivement les énantiomères de l'alcool de formule IV ou du composé hémiacétalique de formule III mis en jeu.

17. Application selon la revendication 16, caractérisée en ce que l'on clive les diastéréoisomères résultant de l'action d'un composé hémiacétalique de formule III et d'un alcool de formule IV choisi dans le groupe constitué par :
l'alcool (RS) α-cyano 3-phénoxy benzylique,
l'alcool (RS) α-cyano 4-fluoro 3-phénoxy benzylique,
l'alcool (RS) cyano 6-phénoxy 2-pyridyl méthylique.

18. Application selon la revendication 16, caractérisée en ce que l'on clive les diastéréoisomères résultant de l'action d'un composé hémiacétalique de formule III et de la (RS) alléthrolone.

19. Les produits suivants résultant de l'application selon la revendication 16 :
l'alcool (S) cyano(6-phénoxy 2-pyridyl) méthylique,
l'alcool (R) cyano(6-phénoxy 2-pyridyl) méthylique.

20. A titre de produits chimiques nouveaux, sous toutes les formes isomères possibles, les hémiacétals de formule générale $III_a$ :

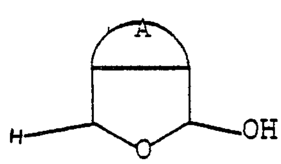

($III_a$)

dans laquelle la chaîne A a pour structure :
soit

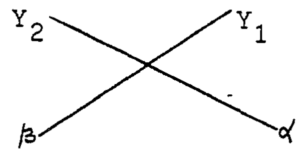

$Y_1$ et $Y_2$, identiques ou différents, représentant un atome de fluor, de chlore ou de brome ou un radical alcoyle comportant de 1 à 6 atomes de carbone ou $Y_1$ et $Y_2$ formant ensemble avec l'atome de carbone auquel ils sont liés, un homocycle carboné comportant de 3 à 7 atomes de carbone ;
soit

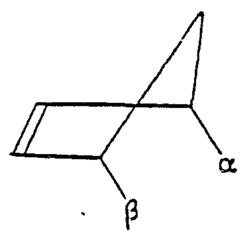

soit

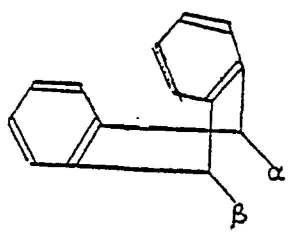

soit

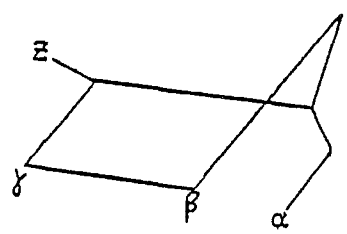

formule dans laquelle Z représente un atome d'hydrogène, un atome de chlore, un atome de brome ou d'iode.

21. A titre de produits chimiques nouveaux, sous toutes les formes isomères possibles, les hémiacétals de formule générale $III_b$ :

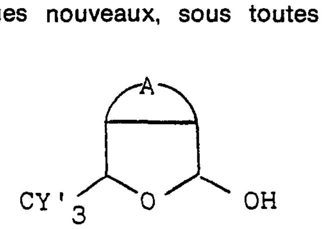

($III_b$)

dans laquelle Y' et A conservent la même signification que dans la revendication 1.

22. A titre de produits chimiques nouveaux, les hémiacétals de formule générale III dont les noms suivent :

le (1R,2S,5S) 6,6-diméthyl 3-oxa bicyclo(3.1.0) hexan-2-ol et sa forme antipodale,

le (1R,2S,4R,5S) 6,6-diméthyl 4-[2-méthyl 2-propyl] 3-oxa bicyclo(3.1.0) hexan-2-ol, et sa forme antipodale,

le (1R,2S,4R,5S) 6,6-diméthyl 4-trichlorométhyl 3-oxa bicyclo(3.1.0) hexan-2-ol et sa forme antipodale,

le (1R,2S,4R,5S) 6,6-diméthyl 4-tribromométhyl 3-oxa(3.1.0) hexan-2-ol et sa forme antipodale,

le (3aR,4R,7S,7aS,1S) tétrahydro 4,7-méthano isobenzofuran-1-ol et sa forme antipodale,

le 9,10,11,12,14,15-hexahydro 9,10 [3',4'] furano anthracène 12-ol racémique,

le (11R,12R,15S) 9,10,11,12,14,15-hexahydro 9,10 [3',4'] furano anthracène-12-ol et sa forme antipodale,

le 6-bromohexahydro 3,5-méthano 2H-cyclopenta [b] furan-2-ol racémique,

le (2S,3S,3aR,5S,6S,6aS) 6-bromohexahydro 3,5-méthano 2H-cyclopenta [b] furan-2-ol et sa forme antipodale,

le (2S,3R,3aR,5S,6aR) hexahydro 3,5-méthano 2H-cyclopenta [b] furan-2-ol et sa forme antipodale.

**Revendications** (pour l'Etat contractant AT)

1. Procédé de dédoublement des composés hémiacétaliques de formule III :

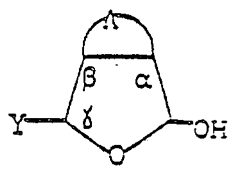

(III)

formule dans laquelle le symbole A représente une chaîne hydrocarbonée comportant de 1 à 16 chaînons, cette chaîne pouvant comporter un ou plusieurs hétéroatomes, une ou plusieurs insaturations, l'ensemble des chaînons constitutifs de la chaîne pouvant représenter un système mono ou polycyclique, y compris un système du type spiro ou endo, l'ensemble constitué par la chaîne A et les atomes de carbone auxquels elle est liée, pouvant comporter un ou plusieurs atomes chiraux ou bien la copule hémiacétalique pouvant présenter une chiralité due à la configuration spatiale dissymétrique de l'ensemble de la molécule, Y représente soit un atome d'hydrogène, soit un groupement $-CY'_3$ dans lequel Y' est un atome de chlore ou de brome, soit un groupement alcoyle linéaire ou ramifié comportant de 1 à 18 atomes de carbone éventuellement substitué, la liaison βγ de la formule I ainsi que le substituant Y pouvant dans ce dernier cas faire partie du substituant A, ainsi que des alcools de formule IV :

R—OH (IV)

formule dans laquelle R représente soit un reste d'alcool primaire, secondaire ou tertiaire, comportant au moins un carbone asymétrique, soit un reste alcoolique substitué dont la chiralité est due à la configuration spatiale dissymétrique de l'ensemble de la molécule, caractérisé en ce que l'on fait réagir l'hydrure de diisobutyl aluminium au sein d'un solvant organique sur un composé racémique ou optiquement actif, de formule générale II :

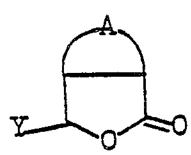

(II)

dans laquelle A et Y conservent les significations précitées, puis soumet le composé de formule générale III résultant :

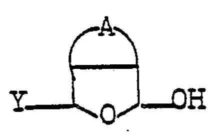

(III)

respectivement à l'action d'un alcool optiquement actif ou racémique de formule générale IV :

R—OH (IV)

formule dans laquelle R conserve les significations précitées pour obtenir un composé de formule I :

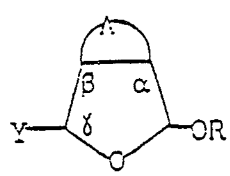

(I)

dans laquelle Y, A et R conservent les significations précitées, dont on sépare les diastéréoisomères, que l'on clive pour obtenir les énantiomères du composé hémiacétalique de formule III, dans le cas où l'on a utilisé au départ un composé hémiacétalique racémique, ou de l'alcool de formule IV, dans le cas où l'on a utilisé au départ un alcool racémique, mis en jeu.

2. Procédé selon la revendication 1, utilisable lorsque Y représente un atome d'hydrogène, caractérisé en ce que l'on fait réagir un hydroborure alcalin au sein d'un solvant, puis un acide au sein d'un solvant organique, avec un composé de formule générale V :

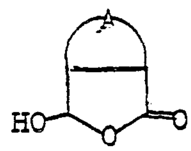

(V)

dans laquelle A conserve la signification précédente, pour obtenir le composé de formule générale $II_A$ :

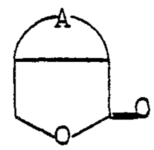

($II_A$)

que l'on traite selon le procédé de la revendication 1.

3. Procédé selon la revendication 1 ou 2, caractérisé en ce que les atomes ou radicaux différents qui substituent le ou les atomes de carbone portés par la chaîne A ou situés en α ou β, sont choisis indifféremment dans l'un ou l'autre des groupes suivants :

a) le groupe constitué par l'hydrogène, les atomes d'halogènes, le groupement nitro, les radicaux alcoyles comportant de 1 à 18 atomes de carbone, les radicaux cyclo alcoyles comportant de 3 à 6 atomes de carbone, le radical phényle, les radicaux phényles substitués soit par un ou plusieurs atomes d'halogènes, soit par un ou plusieurs radicaux alcoyles comportant de 1 à 6 atomes de carbone,

b) le groupe constitué par les radicaux :

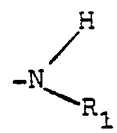

dans lesquels $R_1$ représente un atome d'hydrogène, un radical alcoyle comportant de 1 à 6 atomes de carbone, le radical :

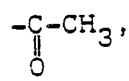

le radical :

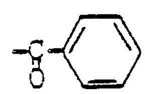

ou le radical :

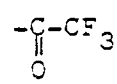

;

un radical alcoyle comportant de 1 à 6 atomes de carbone ou bien dans lesquels $R_2$ et $R_3$ représentent ensemble avec l'atome d'azote auquel ils sont liés, un hétérocycle comportant 6 atomes.

4. Procédé selon la revendication 1, 2 ou 3, caractérisé en ce que la chaîne A a pour structure :

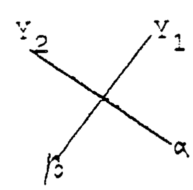

$Y_1$ et $Y_2$, identiques ou différents, représentant un atome d'hydrogène, de fluor, de chlore ou de brome ou un radical alcoyle comportant de 1 à 6 atomes de carbone ou $Y_1$ et $Y_2$ formant ensemble avec l'atome de carbone auquel ils sont liés, un homocycle carboné comportant de 3 à 7 atomes de carbone.

5. Procédé selon la revendication 1, 2, 3 ou 4, caractérisé en ce que la chaîne A a pour structure :

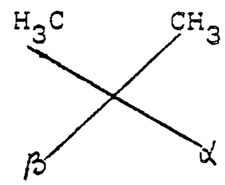

6. Procédé selon la revendication 1, 2 ou 3, caractérisé en ce que la chaîne A a une structure choisie dans le groupe constitué par la chaîne :

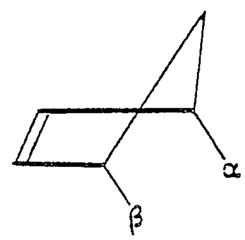

la chaîne :

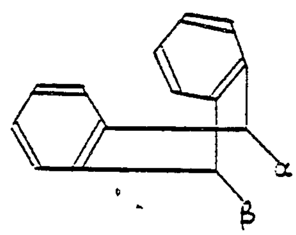

et la chaîne :

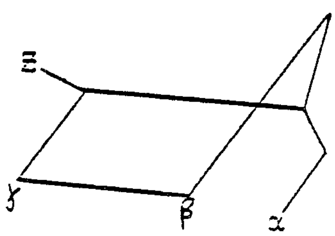

7. Procédé selon l'une quelconque des revendications 1 à 6 caractérisé en ce que le substituant R représente un reste cyanométhyle substitué, choisi dans le groupe constitué par les radicaux :

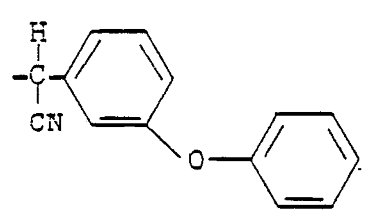

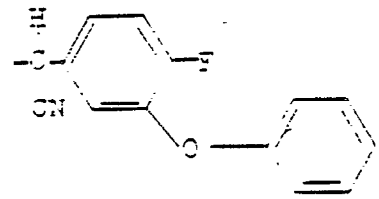

et

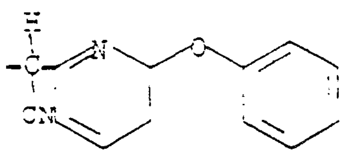

ainsi que par les radicaux correspondants, dans lesquels le groupement cyano est remplacé par un radical alkyle, alkényle ou alkynyle.

8. Procédé selon l'une quelconque des revendications 1 à 6, caractérisé en ce que le substituant R a pour formule :

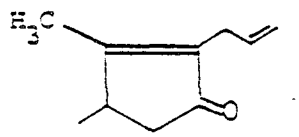

9. Procédé selon l'une quelconque des revendications 1 à 7, caractérisé en ce que l'alcool de formule IV est choisi dans le groupe constitué par :

l'alcool (RS) α-cyano 3-phénoxy benzylique,
l'alcool (RS) α-cyano 4-fluoro 3-phénoxy benzylique,
l'alcool (RS) -cyano 6-phénoxy 2-pyridyl méthylique.

10. Procédé selon l'une quelconque des revendications 1 à 6 et 8, caractérisé en ce que l'alcool de formule IV est la (RS) alléthrolone.

**Claims** (for the contracting States : BE, CH, DE, FR, GB, IT, LI, NL, SE)

1. In all their possible isomeric forms, the compounds with the general formula (I) :

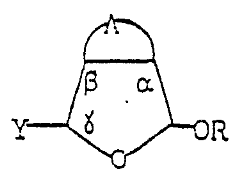

(I)

in which formula the symbol A represents a hydrocarbonated chain containing from 1 to 16 links, this chain being able to contain one or more heteroatoms, one or more unsaturations, the whole of the constituent links of the chain being able to represent a mono or polycyclic system, including a spiro or endo type system, the whole constituted by the chain A and the carbon atoms to which it is linked, being able to contain one or more chiral atoms or the hemiacetal copula being able to present a chirality due to the asymmetric spatial configuration of the whole of the molecule, R represents either a primary, secondary or tertiary alcohol residue, containing at least one asymmetric carbon, or a substituted alcohol residue whose chirality is due to the asymmetric spatial configuration of the whole of the molecule, Y represents either a hydrogen atom, or a —$CY'_3$ group in which Y' is a chlorine or bromine atom, or a linear or branched alkyl group containing from 1 to 18 carbon atoms, possibly substituted, the βγ bond of the formula I as well as substituent Y being able in this last case to form a part of substituent A, as well as the mixtures of these isomers.

2. Compounds according to formula (I) of claim 1, in which the various atoms or radicals which substitute the carbon atom or atoms carried by chain A or situated in α or β, are indifferently chosen from one or other of the following groups :

a) the group constituted by hydrogen, halogen atoms, the nitro group, alkyl radicals containing from 1 to 18 carbons atoms, cyclo alkyl radicals containing from 3 to 6 carbon atoms, a phenyl radical, phenyl radicals substituted either by one or more halogen atoms, or by one or more alkyl radicals containing from 1 to 6 carbon atoms,

b) the group constituted by radicals :

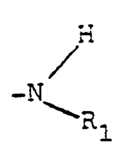

in which $R_1$ represents a hydrogen atom, an alkyl radical containing from 1 to 6 carbon atoms, the radical :

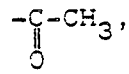

the radical :

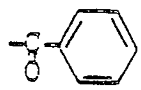

or the radical :

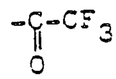

c) the group constituted by radicals :

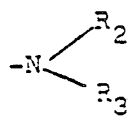

in which $R_2$ and $R_3$, identical or different, each represents an alkyl radical containing from 1 to 6 carbon atoms or in which $R_2$ and $R_3$ represent, together with the nitrogen atom to which they are linked, a heterocycle containing 6 atoms.

3. Compounds according to claim 1 or 2, characterized in that chain A has as its structure :

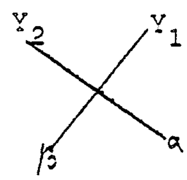

$Y_1$ and $Y_2$, identical or different, each representing a hydrogen, fluorine, chlorine or bromine atom or an alkyl radical containing from 1 to 6 carbon atoms or $Y_1$ and $Y_2$ forming, together with the carbon atom to which they are linked, a carbonated homocycle containing from 3 to 7 carbon atoms.

4. Compounds according to claim 1, 2 or 3, characterized in that chain A has as its structure :

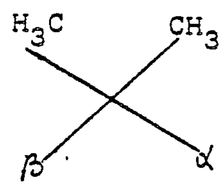

5. Compounds according to claim 1 or 2, characterized in that chain A has as its structure :

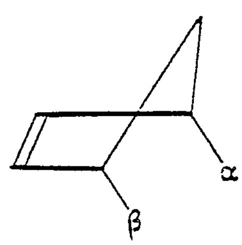

6. Compounds according to claim 1 or 2, characterized in that chain A has as its structure :

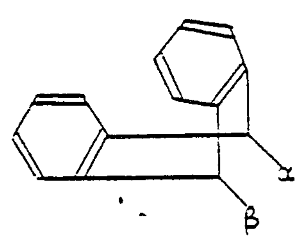

7. Compounds according to claim 1 or 2, characterized in that chain A has as its structure :

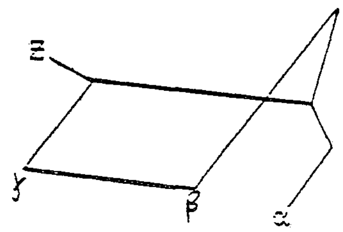

in which formula Z represents a hydrogen atom, a chlorine atom, a bromine or iodine atom.

8. Compounds according to any one of the claims 1 to 7, characterized in that the substituent R represents a substituted cyanomethyl residue, chosen from the group constituted by the radicals :

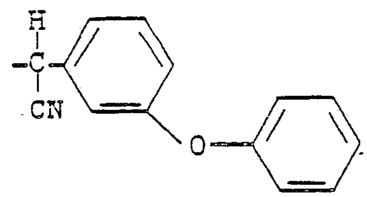

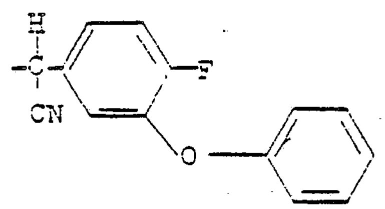

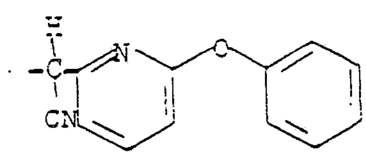

,

as well as by the corresponding radicals, in which the cyano group is replaced by an alkyl, alkenyl or alkynyl radical.

9. Compounds according to any one of the claims 1 to 7, characterized in that the substituent R has as its formula :

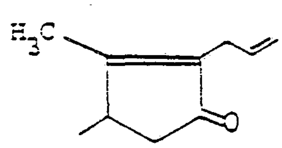

10. Any one of the compounds with the general formula (I), according to claim 1, of which the names follow :

(1R,2R,4R,5S) 6,6-diméthyl 3-oxa 2-[(S) cyano(3-phenoxyphenyl) methoxy] 4-(2-methyl 2-propyl) bicyclo(3,1,0) hexane ;

(1R,2R,4R,5S) 6,6-dimethyl 3-oxa 2-[(R) cyano(3-phenoxyphenyl) methoxy] 4-(2-methyl 2-propyl) bicyclo(3,1,0) hexane ;

(1R,2S,4R,5S) 6,6-dimethyl 4-trichloromethyl 2-[(R) 2-methyl 4-oxo 3-(2-propen-1-yl) cyclopent-2-enyloxy] 3-oxabicyclo(3,1,0) hexane ;

(1R,2S,4R,5S) 6,6-dimethyl 4-trichloromethyl 2-[(S) 2-methyl 4-oxo 3-(2-propen-1-yl) cyclopent-2-enyloxy] 3-oxabicyclo(3,1,0) hexane ;

(1R,2R,5S) 6,6-dimethyl 3-oxa 2-[(S) cyano(3-phenoxyphenyl) methoxy] bicyclo(3,1,0) hexane ;

(1R,2R,5S) 6,6-dimethyl 3-oxa 2-[(R) cyano(3-phenoxyphenyl) methoxy] bicyclo(3,1,0) hexane ;

(1R,2S,4R,5S) 6,6-dimethyl 4-trichloromethyl 2-[(S) cyano(3-phenoxyphenyl) methoxy] 3-oxa bicyclo(3,1,0) hexane ;

(1R,2S,4R,5S) 6,6-dimethyl 4-trichloromethyl 2-[(R) cyano(3-phenoxyphenyl) methoxy] 3-oxa bicyclo(3,1,0) hexane ;

(1R,2S,4R,5S) 6,6-dimethyl 4-tribromomethyl 2-[(R) 2-methyl 4-oxo 3-(2-propen-1-yl) cyclopent-2-enyloxy] 3-oxa bicyclo(3,1,0) hexane ;

(1R,2S,4R,5S) 6,6-dimethyl 4-tribromomethyl 2-[(S) 2-methyl 4-oxo 3-(2-propen-1-yl) cyclopent-2-enyloxy] 3-oxa bicyclo(3,1,0) hexane ;

(1R,2R,5S) 6,6-dimethyl 3-oxa 2-[(R) cyano(3-phenoxy 4-fluorophenyl) methoxy] bicyclo(3,1,0) hexane ;

(1R,2R,5S) 6,6-dimethyl 3-oxa 2-[(S) cyano(3-phenoxy 4-fluorophenyl) methoxy] bicyclo(3,1,0) hexane ;

(1R,2R,5S) 6,6-dimethyl 3-oxa 2-[(S) cyano(6-phenoxy 2-pyridinyl) methoxy] bicyclo(3,1,0) hexane ;

(1R,2R,5S) 6,6-dimethyl 3-oxa 2-[(R) cyano(6-phenoxy-2-pyridinyl) methoxy] bicyclo(3,1,0) hexane.

11. Any one of the compounds with the general formula (I), according to claim 1, of which the names follow :

(3aR,4R,7S,7aS,1R) 1-[(R) cyano(3-phenoxyphenyl) methoxy] tetrahydro-4,7-methano-isobenzofuran ;

(3aR,4R,7S,7aS,12) 1-[(S) cyano(3-phenoxyphenyl) methoxy] tetrahydro-4,7-methano-isobenzofuran.

12. Any one of the compounds with the general formula (I), according to claim 1, of which the names follow :

(11S,12R,15R) 12-[(S) cyano(3-phenoxyphenyl) methoxy] 9,10,11,12,14,15-hexahydro 9,10 [3',4'] furano anthracene ;

(11R,12S,15S) 12-[(S) cyano(3-phenoxyphenyl) methoxy] 9,10,11,12,14,15-hexahydro 9,10 [3',4'] furano anthracene ;

(11R,12S,15S) 12-[(1S) 2-methyl 4-oxo 3-(2-propen-1-yl) cyclopent-2-enyloxy] 9,10,11,12,14,15-hexahydro 9,10 [3',4'] furano anthracene ;

(11R,12S,15S) 12-[(1R) 2-methyl 4-oxo 3-(2-propen-1-yl) cyclopent-2-enyloxy] 9,10,11,12,14,15-hexahydro 9,10 [3',4'] furano anthracene ;

(11R,12S,15S) 12-[(R) (4,4-dimethyl 2-oxo tetrahydrofuran-3-yl) oxy] 9,10,11,12,14,15-hexahydro 9,10 [3',4'] furano anthracene ;

(11R,12S,15S) 12-[(S) (4,4-dimehyl 2-oxo tetrahydrofuran-3-yl) oxy] 9,10,11,12,14,15-hexahydro 9,10 [3',4'] furano anthracene ;

(11S,12R,15S) 12-[(1R,2S,5R) 2-prop-2-yl 5-methylcyclohexyloxy] 9,10,11,12,14,15-hexahydro 9,10 [3',4'] furano anthracene ;

(11R,12S,15S) 12-[(1R,2S,5R) 2-prop-2-yl 5-methylcyclohexyloxy] 9,10,11,12,14,15-hexahydro 9,10 [3',4'] furano anthracene.

13. Any one of the compounds with the general formula (I), according to claim 1, of which the names follow :

[(12R) (6-bromohexahydro 3,5-methano 2H-cyclopenta [b] furan-2-yl) oxy] (S) α-cyano 3-phenoxybenzyl,

[(2S) (6-bromohexahydro 3,5-methano 2H-cyclopenta [b] furan-2-yl) oxy] (S) α-cyano 3-phenoxybenzyl.

14. Preparation process for compounds with the general formula (I) as defined in claim 1, characterized in that diisobutyl aluminium hydride is made to react, in an organic solvent, on a racemic or optically active compound with the general formula (II) :

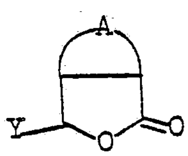

(II)

in which A and Y keep the previously given significances, then the resulting compound with the general formula (III) :

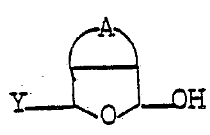

(III)

is submitted to the action of a racemic or optically active alcohol with the general formula (IV) :

R—OH (IV)

in which formula R keeps the previously given significances, so as to obtain the corresponding compound with the formula (I), the diastereoisomers of which are, if necessary or desired, separated.

15. Process according to claim 14, usable when Y represents a hydrogen atom, characterized in that an alkali borohydride, in a solvent, then an acid, in an organic solvent, are made to react with a compound with the general formula (V) :

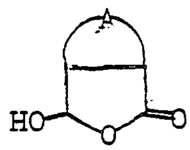

(V)

in which A keeps the previous significance, so as to obtain the compound with the general formula ($II_A$) :

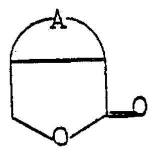

($II_A$)

which is treated according to the process of claim 11.

16. Use of compounds with the general formula (I) as defined in claim 1 in the resolution of compounds with the general formula (III) or (IV), which use consists of :
either making an optically active hemiacetal compound with the general formula (III) :

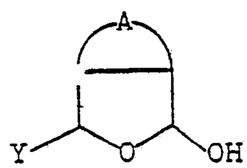

(III)

in which Y and A keep their previously given significances, react with a racemic alcohol with the general formula (IV) :

R—OH (IV)

in which R keeps its previous significance,
or making a racemic hemiacetal compound with the general formula (III) :

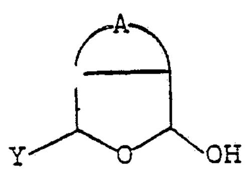

(III)

in which Y and A keep their previous significances, react with an optically active alcohol with the general formula (IV) :

R—OH (IV)

in which R keeps is previous significance, so as to obtain a mixture of diastereoisomers with the formula (I) which is separated according to traditional processes, which use is characterized in that the said diastereoisomers with the formula (I) are cloaved, so as to obtain respectively the enantiomers of the alcohol with the formula (IV), or of the hemiacetal compound with the formula (III), brought into operation.

17. Use according to claim 16, characterized in that the diastereoisomers are cleaved which result from the action of a hemiacetal compound with the formula (III) and of an alcohol with the formula (IV) chosen from the group constituted by :

(RS) α-cyano-3-phenoxybenzyl alcohol,
(RS) α-cyano-4-fluoro-3-phenoxybenzyl alcohol,
(RS) -cyano-6-phenoxy-2-pyridyl-methyl alcohol.

18. Use according to claim 16, characterized in that the resulting diastereoisomers are cleaved, which result from the action of a hemiacetal compound with the formula (III) and of (RS) allethrolone.

19. The following resulting products from the use according to claim 16 :

(S) cyano(6-phenoxy-2-pyridyl) methyl alcohol,

(R) cyano(6-phenoxy-2-pyridyl) methyl alcohol.

20. As new chemical products, in all the possible isomeric forms, hemiacetals with the general formula ($III_a$) :

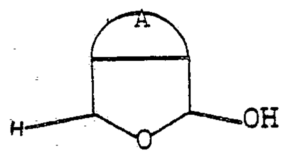

($III_a$)

in which chain A has as its structure :
either

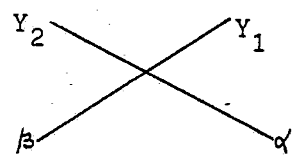

$Y_1$ and $Y_2$, identical or different, representing a fluorine, chlorine or bromine atom or an alkyl radical containing from 1 to 6 carbon atoms or $Y_1$ and $Y_2$ forming, together with the carbon atom to which they are linked, a carbonated homocycle containing from 3 to 7 carbon atoms ;
or

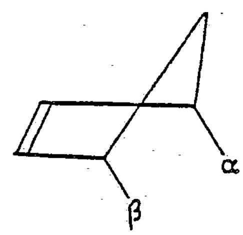

or

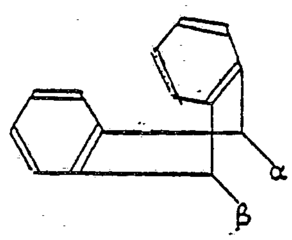

or

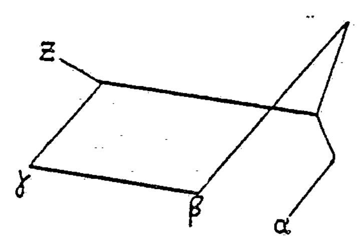

in which formula Z represents a hydrogen atom, a chlorine atom, a bromine or iodine atom.

21. As new chemical products, in all the possible isomeric forms, hemiacetals with the general formula ($III_b$) :

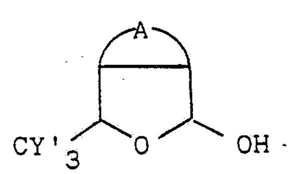

($III_b$)

in which Y′ and A keep the same significances as in claim 1.

22. As new chemical products, hemiacetals with the general formula (III) of which the names follow :

(1R,2S,5S) 6,6-dimethyl-3-oxabicyclo [3,1,0] hexan-2-ol and its antipodal form,

(1R,2S,4R,5S) 6,6-dimethyl-4-[2-methyl-2-propyl]-3-oxabicyclo [3,1,0] hexan-2-ol and its antipodal form,

(1R,2S,4R,5S) 6,6-dimethyl-4-trichloromethyl-3-oxabicyclo [3,1,0] hexan-2-ol and its antipodal form,

(1R,2S,4R,5S) 6,6-dimethyl-4-tribromomethyl-3-oxa [3,1,0] hexan-2-ol and its antipodal form,

(1R,2S,4R,5S) 6,6-dimethyl-4-tribromomethyl-3-oxa [3,1,0] hexan-2-ol and its and podal form,

(3aR,4R,7S,7aS,1S) tetrahydro-4,7-methano-isobenzofuran-1-ol and its antipodal form,

racemic 9,10,11,12,14,15-hexahydro-9,10 [3′,4′] furanoanthracen-12-ol,

(11R,12R,15S) 9,10,11,12,14,15-hexahydro-9,10 [3′,4′] furanoanthracen-12-ol and its antipodal form,

racemic 6-bromo-hexahydro-3,5-methano-2H-cyclopenta [b] furan-2-ol,

(2S,3S,3aR,5S,6S,6aS) 6-bromo-hexahydro-3,5-methano-2H-cyclopenta [b] furan-2-ol and its antipodal form,

(2S,3S,3aR,5S,6aR) hexahydro-3,5-methano-2H-cyclopenta [b] furan-2-ol and its antipodal form.

**Claims** (for the Contracting State AT)

1. Resolution process for hemiacetal compound with the formula (III) :

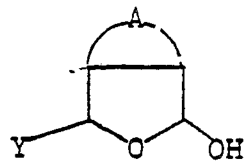

(III)

in which formula the symbol A represents a hydrocarbonated chain containing from 1 to 16 links, this chain being able to contain one or more heteroatoms, one or more unsaturations, the whole of the constituent links of the chain being able to represent a mono or polycyclic system, including a spiro or endo type system, the whole constituted by the chain A and the carbon atoms to which it is linked, being able to contain one or more chiral atoms or the hemiacetal copula being able to present a chirality due to the asymmetric spatial configuration of the whole of the molecule, Y represents either a hydrogen atom, or a $—CY'_3$ group in which Y′ is a chlorine or bromine atom, or a linear or branched alkyl group containing from 1 to 18 carbon atoms, possibly substituted the βγ bond of the formula I as well as substituent Y being able in this case to form a part of substituent A, as well as the alcohols with the formula (IV) :

$$R—OH \qquad (IV)$$

in which formula R represents either a primary, secondary or tertiary alcohol residue, containing at least one asymmetric carbon, or a substituted alcohol residue whose chirality is due to the asymmetric spatial configuration of the whole of the molecule, characterized in that diisobutyl aluminium hydride is made to react, in an organic solvent, on a racemic or optically active compound with the general formula (II) :

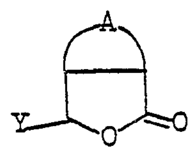

(II)

in which A and Y keep the previously given significances, then the resulting compound with the general formula (III) :

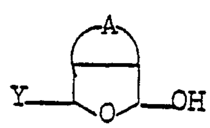

(III)

is submitted respectively to the action of an optically active or racemic alcohol with the general formula (IV) :

$$R—OH \qquad (IV)$$

in which formula R keeps the previously given significances, so as to obtain a compound with the formula (I) :

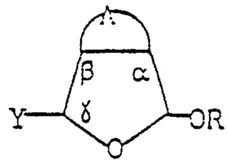

(I)

in which Y, A and R keep the previously given significances, of which the diastereoisomers are separated and are cleaved so as to obtain the enantiomers either of the hemiacetal compound with the formula (III) which is made to react, in the case where at the start a racemic hemiacetal compound has been used, or of the alcohol with the formula (IV) which is made to react, in the case where at the start a racemic alcohol has been used.

2. Process according to claim 1, usable when Y represents a hydrogen atom, characterized in that an alkali borohydride in a solvent, then an acid in an organic solvent, are made to react with a compound with the general formula (V) :

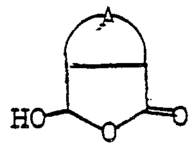

(V)

in which A keeps the previous significance, so as to obtain the compound with the general formula ($II_A$) :

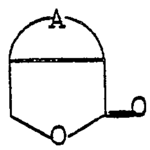

($II_A$)

which is treated according to the process of claim 1.

3. Process according to claim 1 or 2, characterized in that the various atoms or radicals which substitute the carbon atom or atoms carried by chain A or situated in α or β, are chosen indifferently from one or other of the following groups :

a) the group constituted by hydrogen, halogen atoms, the nitro group, alkyl radicals containing from 1 to 18 carbon atoms, cyclo alkyl radicals containing from 3 to 6 carbon atoms, a phenyl radical, phenyl radicals substituted either by one or more halogen atoms, or by one or more alkyl radicals containing from 1 to 6 carbon atoms,

b) the group constituted by radicals :

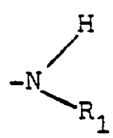

in which $R_1$ represents a hydrogen atom, an alkyl radical containing from 1 to 6 carbon atoms, the radical :

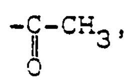

the radical

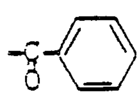

or the radical :

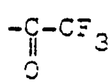

c) the group constituted by the radicals

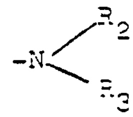

in which $R_2$ and $R_3$, identical or different, each represents an alkyl radical containing from 1 to 6 carbon atoms or in which $R_2$ and $R_3$ represent, together with the nitrogen atom to which they are linked, a heterocycle containing 6 atoms.

4. Process according to claim 1, 2 or 3, characterized in that chain A has as its structure :

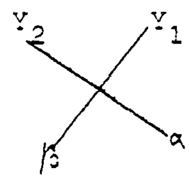

$Y_1$ and $Y_2$, identical or different, each representing a hydrogen, fluorine, chlorine or bromine atom or an alkyl radical containing from 1 to 6 carbon atoms or $Y_1$ and $Y_2$ forming, together with the carbon atom to which they are linked, a carbonated homocycle containg from 3 to 7 carbon atoms.

5. Process according to claim 1, 2, 3 or 4, characterized in that chain A has as its structure :

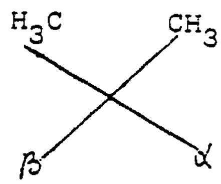

6. Process according to claim 1, 2 or 3, characterized in that chain A has a structure chosen from the group constituted by the chain :

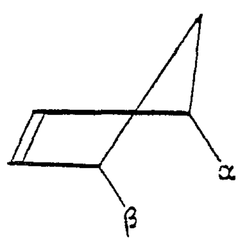

the chain :

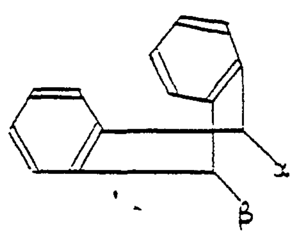

and the chain :

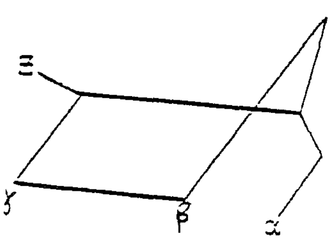

7. Process according to any one of the claims 1 to 6 characterized in that substituent R represents substituted cyanomethyl residue, chosen from the group constituted by radicals :

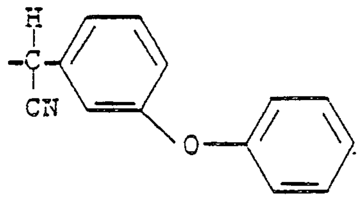

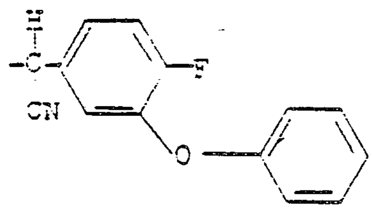

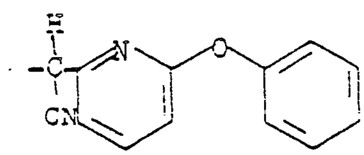

as well as by the corresponding radicals, in which the cyano group is replaced by an alkyl, alkenyl or alkynyl radical.

8. Process according to any one of the claims 1 to 6, characterized in that substituent R has as its formula :

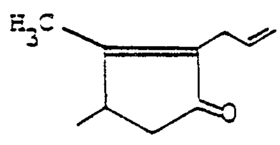

9. Process according to any one of the claims 1 to 7, characterized in that the alcohol with the formula (IV) is chosen from the group constituted by :

(RS) α-cyano-3-phenoxybenzyl alcohol,

(RS) α-cyano-4-fluoro-3-phenoxybenzyl alcohol,

(RS)-cyano-6-phenoxy-2-pyridylmethyl alcohol.

10. Process according to any one of the claims 1 to 6 and 8, characterized in that the alcohol with the formula (IV) is (RS) allethrolone.

**Patentansprüche** (für die Vertragsstaaten : BE, CH, DE, FR, GB, IT, LI, LU, NL, SE)

1. In sämtlichen ihrer möglichen isomeren Formen die Verbindungen der allgemeinen Formel (I)

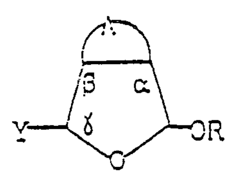

(I)

worin das Symbol A eine Kohlenwasserstoffkette mit 1 bis 16 Kettengliedern bedeutet, wobei diese Kette ein oder mehrere Heteroatome, eine oder mehrere Unsättigungen enthalten kann und die Gesamtheit der die Kette bildenden Kettenglieder ein mono- oder polycyclisches System einschließlich eines Systems

vom spiro- oder endo-Typ darstellen kann, die durch die Kette A und die Kohlenstoffatome, an die sie gebunden ist, gebildete Gesamtheit ein oder mehrere chirale Atome oder die hemiacetalische Verknüpfung enthalten kann, die aufgrund der asymmetrischen räumlichen Konfiguration der Gesamtheit des Moleküls eine Chiralität aufweisen kann, R entweder einen Rest eines primären, sekundären oder tertiären Alkohols, der zumindest ein asymmetrisches Kohlenstoffatom enthält, oder einen Rest eines substituierten Alkohols darstellt, dessen Chiralität auf die asymmetrische räumliche Konfiguration der Gesamtheit des Moleküls zurückzuführen ist, Y entweder ein Wasserstoffatom oder eine Gruppe $-CY'_3$, worin Y' ein Chlor- oder Bromatom bedeutet, oder eine gegebenenfalls substituierte, lineare oder verzweigte Alkylgruppe mit 1 bis 18 Kohlenstoffatomen darstellt, wobei die βα-Bindung der Formel I ebenso wie der Substituent Y in diesem letztgenannten Fall einen Teil des Substituenten A bilden, sowie die Gemische dieser Isomeren.

2. Verbindungen der Formel I gemäß Anspruch 1, worin die verschiedenen Atome oder Reste, die das oder die Kohlenstoffatome substituieren, die von der Kette A getragen werden oder sich in α- oder β-Stellung befinden, ohne Unterschied aus der einen oder anderen der folgenden Gruppen ausgewählt sind :

a) der Gruppe, bestehend aus Wasserstoff, den Halogenatomen, der Nitrogruppe, den Alkylresten mit 1 bis 18 Kohlenstoffatomen, den Cycloalkylresten mit 3 bis 6 Kohlenstoffatomen, der Phenylgruppe, den Phenylresten, die entweder durch ein oder mehrere Halogenatome oder durch einen oder mehrere Alkylreste mit 1 bis 6 Kohlenstoffatomen substituiert sind ;

b) der Gruppe, bestehend aus den Resten

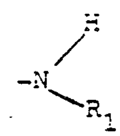

worin die Gruppen $R_1$ ein Wasserstoffatomen, einen Alkylrest mit 1 bis 6 Kohlenstoffatomen, den Rest

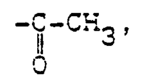

den Rest

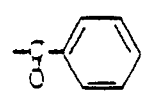

oder den Rest

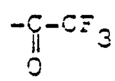

bedeuten ;

c) der Gruppe, bestehend aus den Resten

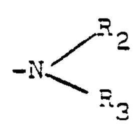

worin $R_2$ und $R_3$, die gleich oder voneinander verschieden sind, einen Alkylrest mit 1 bis 6 Kohlenstoffatomen darstellen oder die Reste $R_2$ und $R_3$ gemeinsam mit dem Stickstoffatom, an das sie gebunden sind, einen Heterocyclus mit 6 Atomen bilden.

3. Verbindungen gemäß Anspruch 1 oder 2, dadurch gekennzeichnet, daß die Kette A die folgende Struktur

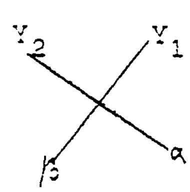

besitzt, worin $Y_1$ und $Y_2$, die gleich oder voneinander verschieden sind, ein Wasserstoff-, Fluor-, Chlor- oder Bromatom oder einen Alkylrest mit 1 bis 6 Kohlenstoffatomen bedeuten oder $Y_1$ und $Y_2$ gemeinsam mit dem Kohlenstoffatom, an das sie gebunden sind, einen Kohlenstoff-Homocyclus mit 3 bis 7 Kohlenstoffatomen bilden.

4. Verbindungen gemäß Anspruch 1, 2 oder 3, dadurch gekennzeichnet, daß die Kette A die folgende Struktur besitzt :

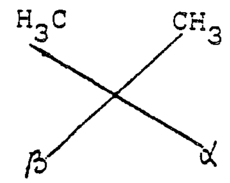

5. Verbindungen gemäß Anspruch 1 oder 2, dadurch gekennzeichnet, daß die Kette A die folgende Struktur besitzt :

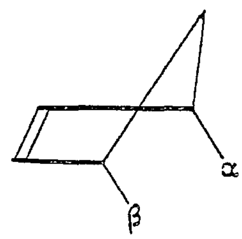

6. Verbindungen gemäß Anspruch 1 oder 2, dadurch gekennzeichnet, daß die Kette A die folgende Struktur besitzt :

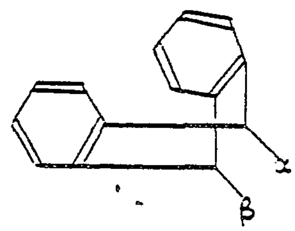

7. Verbindungen gemäß Anspruch 1 oder 2, dadurch gekennzeichnet, daß die Kette A die folgende Struktur besitzt :

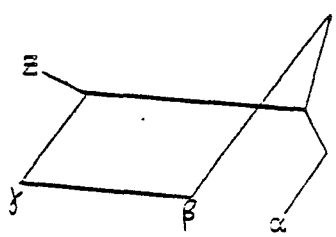

worin Z ein Wasserstoffatom, ein Chlor-, Brom- oder Jodatom bedeutet.

8. Verbindungen gemäß einem der Ansprüche 1 bis 7, dadurch gekennzeichnet, daß der Substituent R einen substituierten Cyanomethylrest bedeutet, ausgewählt unter den Resten

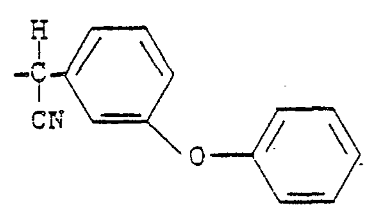

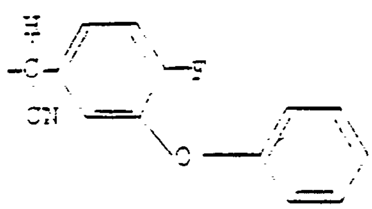

und

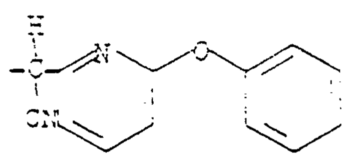
,

sowie den entsprechenden Resten, in denen die Cyanogruppe durch einen Alkyl-, Alkenyl- oder Alkinylrest ersetzt ist.

9. Verbindungen gemäß einem der Ansprüche 1 bis 7, dadurch gekennzeichnet, daß der Substituent R die Formel

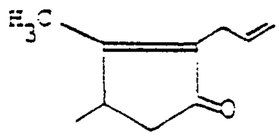
.

besitzt.

10. Eine der Verbindungen der allgemeinen Formel (I) gemäß Anspruch 1 mit den folgenden Bezeichnungen :

(1R,2R,4R,5S)-6,6-Dimethyl-3-oxa-2-[S-cyano-(3-phenoxyphenyl)-methoxy]-4-(2-methyl-2-propyl)-bicyclo [3.1.0] hexan ;

(1R,2R,4R,5S)-6,6-Dimethyl-3-oxa-2-[R-cyano-(3-phenoxyphenyl)-methoxy]-4-(2-methyl-2-propyl)-bicyclo [3.1.0] hexan ;

(1R,2S,4R,5S)-6,6-Dimethyl-4-trichlormethyl-2-[(R)-2-methyl-4-oxo-3-(2-propen-1-yl)-cyclopent-2-enyloxy]-3-oxa-bicyclo [3.1.0] hexan ;

(1R,2S,4R,5S)-6,6-Dimethyl-4-trichlormethyl-2-[(S)-2-methyl-4-oxo-3-(2-propen-1-yl)-cyclopent-2-enyloxy]-3-oxa-bicyclo [3.1.0] hexan ;

(1R,2R,5S)-6,6-Dimethyl-3-oxa-2-[S-cyano-(3-phenoxyphenyl)-methoxy]-bicyclo [3.1.0] hexan ;

(1R,2R,5S)-6,6-Dimethyl-3-oxa-2-[R-cyano-(3-phenoxyphenyl)-methoxy]-bicyclo [3.1.0] hexan ;

(1R,2S,4R,5S)-6,6-Dimethyl-4-trichlormethyl-2-[(S)-cyano-(3-phenoxyphenyl)-methoxy]-3-oxa-bicyclo [3.1.0] hexan ;

(1R,2S,4R,5S)-6,6-Dimethyl-4-trichlormethyl-2-[(R)-cyano-(3-phenoxyphenyl)-methoxy]-3-oxa-bicyclo [3.1.0] hexan ;

(1R,2S,4R,5S)-6,6-Dimethyl-4-tribrommethyl-2-[(R)-2-methyl-4-oxo-3-(2-propen-1-yl)-cyclopent-2-enyloxy]-3-oxa-bicyclo [3.1.0] hexan ;

(1R,2S,4R,5S)-6,6-Dimethyl-4-tribrommethyl-2-[(S)-2-methyl-4-oxo-3-(2-propen-1-yl)-cyclopent-2-enyloxy]-3-oxa-bicyclo [3.1.0] hexan ;

(1R,2R,5S)-6,6-Dimethyl-3-oxa-2-[(R)-cyano-(3-phenoxy-4-fluorphenyl)-methoxy]-bicyclo [3.1.0] hexan ;

(1R,2R,5S)-6,6-Dimethyl-3-oxa-2-[(S)-cyano-(3-phenoxy-4-fluorphenyl)-methoxy]-bicyclo [3.1.0] hexan ;

(1R,2R,5S)-6,6-Dimethyl-3-oxa-2[(S)-cyano-(6-phenoxy-2-pyridinyl)-methoxy]-bicyclo [3.1.0] hexan ;

(1R,2R,5S)-6,6-Dimethyl-3-oxa-2-[(R)-cyano-(6-phenoxy-2-pyridinyl)-methoxy]-bicyclo [3.1.0] hexan.

11. Eine der Verbindungen der allgemeinen Formel (I) gemäß Anspruch 1 mit den folgenden Bezeichnungen :

(3aR,4R,7S,7aS,1R)-1-[R-(Cyano-3-phenoxyphenyl)-methoxy]-tetrahydro-4,7-methano-isobenzofuran ;

(3aR,4R,7S,7aS,12)-1-[S-(Cyano-3-phenoxyphenyl)-methoxy]-tetrahydro-4,7-methano-isobenzofuran.

12. Eine der Verbindungen der allgemeinen Formel (I) gemäß Anspruch 1 mit den folgenden Bezeichnungen :

(11S,12R,15R)-12-[S-(Cyano-3-phenoxyphenyl)-methoxy]-9,10,11,12,14,15-hexahydro-9,10 [3',4'] furanoanthracen ;

(11R,12S,15S)-12-[S-Cyano-3-phenoxyphenyl)-methoxy]-9,10,11,12,14,15-hexahydro-9,10 [3',4'] furanoanthracen ;

(11R,12S,15S)-12-[1S-2-Methyl-4-oxo-3-(2-propen-1-yl)-cyclopent-2-enyloxy]-9,10,11,12,14,15-hexahydro-9,10 [3',4'] furanoanthracen ;

(11R,12S,15S)-12-[1R-2-Methyl-4-oxo-3-(2-propen-1-yl)-cyclopent-2-enyloxy]-9,10,11,12,14,15-hexahydro-9,10 [3',4'] furanoanthracen ;

(11R,12S,15S)-12-[(R)-4,4-Dimethyl-2-oxo-tetrahydrofuran-3-yl)-oxy]-9,10,11,12,14,15-hexahydro-9,10 [3',4'] furanoanthracen ;

(11R,12S,15S)-12-[(S)-(4,4-Dimethyl-2-oxo-tetrahydrofuran-3-yl)-oxy]-9,10,11,12,14,15-hexahydro-9,10 [3',4'] furanoanthracen ;

(11S,12R,15S)-12-[(1R,2S,5R)-2-Prop-2-yl-5-methyl-cyclohexyloxy]-9,10,11,12,14,15-hexahydro-9,10 [3',4'] furanoanthracen ;

(11R,12S,15S)-12-[(1R,2S,5R)-2-Prop-2-yl-5-methyl-cyclohexyloxy]-9,10,11,12,14,15-hexahydro-9,10 [3',4'] furanoanthracen.

13. Eine der Verbindungen der allgemeinen Formel (I) gemäß Anspruch 1 mit den folgenden Bezeichnungen :

12R-(6-Bromhexahydro-3,5-methano-2H-cyclopenta [b] furan-2-yl)-oxy]-(S)-α-cyano-3-phenoxy-benzyl ;

2S-(6-Bromhexahydro-3,5-methano-2H-cyclopenta [b] furan-2-yl)-oxy]-(S)-α-cyano-3-phenoxy-benzyl.

14. Verfahren zur Herstellung der Verbindungen der allgemeinen Formel (I) gemäß Anspruch 1, dadurch gekennzeichnet, daß man ein Diisobutylaluminiumhydrid in dem Medium eines organischen Lösungsmittels mit einer racemischen oder optisch aktiven Verbindung der allgemeinen Formel II

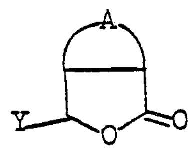

(II)

worin A und Y die angegebenen Bedeutungen besitzen, umsetzt, anschließend die entstandene Verbindung der allgemeinen Formel III

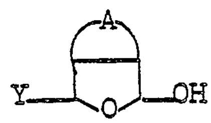

(III)

der Einwirkung eines racemischen oder optisch aktiven Alkohols der allgemeinen Formel IV

$$R—OH \quad (IV)$$

unterzieht, worin R die vorstehend angegebene Bedeutung besitzt, um die entsprechende Verbindung der Formel I zu erhalten, deren Diastereomere man gegebenenfalls trennt.

15. Verfahren gemäß Anspruch 14, das anwendbar ist, wenn Y ein Wasserstoffatom bedeutet, dadurch gekennzeichnet, daß man ein Alkaliborhydrid in dem Medium eines Lösungsmittels, dann eine Säure in dem Medium eines organischen Lösungsmittels mit einer Verbindung der allgemeinen Formel V

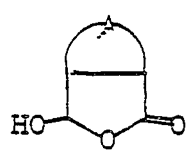

(V)

worin A die vorstehend angegebene Bedeutung besitzt, umsetzt, um die Verbindung der allgemeinen Formel $II_A$

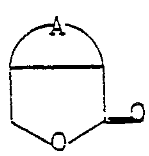

($II_A$)

zu erhalten, die man gemäß dem Verfahren des Anspruchs 11 behandelt.

16. Verwendung der Verbindungen der allgemeinen Formel I gemäß Anspruch 1 zur Spaltung bzw. Auftrennung der Verbindungen der allgemeinen Formel III oder IV, wobei die Verwendung darin besteht, daß man

entweder eine optisch aktive, hemiacetalische Verbindung der allgemeinen Formel III

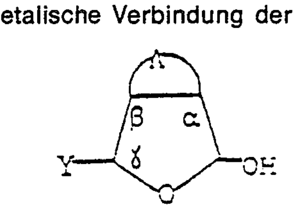

(III)

worin Y und A die vorstehende Bedeutung besitzen, mit einem racemischen Alkohol der allgemeinen Formel IV

R—OH (IV)

umsetzt, worin R die vorstehende Bedeutung besitzt, oder eine racemische, hemiacetalische Verbindung der allgemeinen Formel III

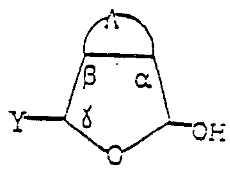

(III)

worin Y und A die vorstehend angegebene Bedeutung besitzen, mit einem optisch aktiven Alkohol der allgemeinen Formel IV

R—OH (IV)

umsetzt, worin R die angegebene Bedeutung besitzt, um ein Gemisch der Diastereomeren der Formel I zu erhalten, das man nach klassischen Methoden auftrennt, wobei die Verwendung dadurch gekennzeichnet ist, daß man die Diastereomeren der Formel I spaltet, um die Enantiomeren des eingesetzten Alkohols der Formel IV bzw. der eingesetzten, hemiacetalischen Verbindung der Formel III zu erhalten.

17. Verwendung gemäß Anspruch 16, dadurch gekennzeichnet, daß man die Diastereomeren spaltet, die der Umsetzung einer hemiacetalischen Verbindung der Formel III und eines Alkohols der Formel IV, ausgewählt unter :

(RS)-α-Cyano-3-phenoxy-benzylalkohol,
(RS)-α-Cyano-4-fluor-3-phenoxy-benzylalkohol,
(RS)-Cyano-6-phenoxy-2-pyridyl-methylalkohol entstammen.

18. Verwendung gemäß Anspruch 16, dadurch gekennzeichnet, daß man die Diastereomeren spaltet, die der Umsetzung einer hemiacetalischen Verbindung der Formel III und des (RS)-Allethrolons entstammen.

19. Die folgenden, der Verwendung gemäß Anspruch 16 entstammenden Produkte :

(S)-Cyano-(6-phenoxy-2-pyridyl)-methylalkohol,
(R)-Cyano-(6-phenoxy-2-pyridyl)-methylalkohol.

20. Als neue, chemische Produkte in sämtlichen ihrer möglichen isomeren Formen die Hemiacetale der allgemeinen Formel $III_a$

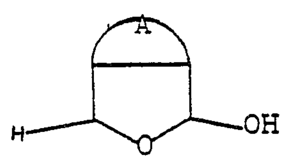

($III_a$)

worin die Kette A eine der folgenden Strukturen besitzt :
entweder

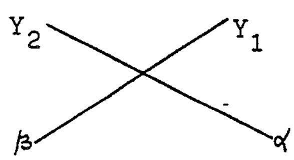

worin $Y_1$ und $Y_2$, die gleich oder voneinander verschieden sind, ein Fluor-, Chlor- oder Bromatom oder einen Alkylrest mit 1 bis 6 Kohlenstoffatomen bedeuten oder $Y_1$ und $Y_2$ gemeinsam mit dem Kohlenstoffatom, an das sie gebunden sind, einen Kohlenstoff-Homocyclus mit 3 bis 7 Kohlenstoffatomen bilden,
oder

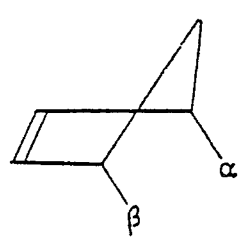

oder

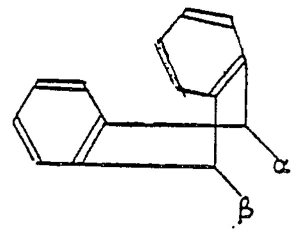

oder

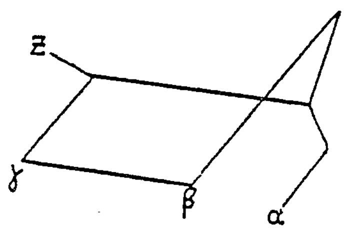

worin Z ein Wasserstoffatom, ein Chlor-, Brom- oder Jodatom bedeutet.

21. Als neue, chemische Produkte in sämtlichen ihren möglichen isomeren Formen die Hemiacetale der allgemeinen Formel $III_b$

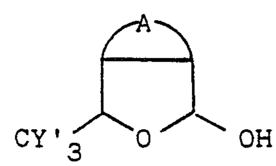

($III_b$)

worin Y' und A die in Anspruch 1 angegebene Bedeutung besitzen.

22. Als neue, chemische Produkte die Hemiacetale der allgemeinen Formel III mit den folgenden Bezeichnungen :

(1R,2S,5S)-6,6-Dimethyl-3-oxa-bicyclo [3.1.0] hexan-2-ol und dessen Antipodenform,

(1R,2S,4R,5S)-6,6-Dimethyl-4-[2-methyl-2-propyl]-3-oxa-bicyclo [3.1.0] hexan-2-ol und dessen Antipodenform,

(1R,2S,4R,5S)-6,6-Dimethyl-4-trichlormethyl-3-oxa-bicyclo [3.1.0] hexan-2-ol und dessen Antipodenform,

(1R,2S,4R,5S)-6,6-Dimethyl-4-tribrommethyl-3-oxa [3.1.0] hexan-2-ol und dessen Antipodenform,

(3aR,4R,7S,7aS,1S)-Tetrahydro-4,7-methano-isobenzofuran-1-ol und dessen Antipodenform,

racemisches 9,10,11,12,14,15-Hexahydro-9,10 [3',4'] furanoanthracen-12-ol,

(11R,12R,15S)-9,10,11,12,14,15-Hexahydro-9,10 [3',4'] furanoanthracen-12-ol und dessen Antipodenform,

racemisches 6-Brom-hexahydro-3,5-methano-2H-cyclopenta [b] furan-2-ol,

(2S,3S,3aR,5S,6S,6aS)-6-Brom-hexahydro-3,5-methano-2H-cyclopenta [b] furan-2-ol, und dessen Antipodenform,

(2S,3R,3aR,5S,6aR)-Hexahydro-3,5-methano-2H-cyclopenta [b] furan-2-ol und dessen Antipodenform.

**Patentansprüche** (für den Vertragsstaat AT)

1. Verfahren zur Spaltung bzw. Auftrennung der hemiacetalischen Verbindungen der Formel III

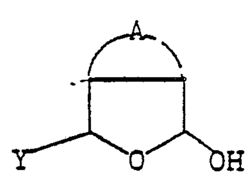

(III)

worin das Symbol A eine Kohlenwasserstoffkette mit 1 bis 16 Kettengliedern darstellt, wobei diese Kette ein oder mehrere Heteroatome, eine oder mehrere Unsättigungen enthalten kann, die Gesamtheit der die Kette bildenden Kettenglieder ein mono- oder polycyclisches System einschließlich eines Systems vom spiro- oder endo-Typ darstellen kann, die durch die Kette A und die Kohlenstoffatome, an die sie gebunden ist, gebildete Gesamtheit ein oder mehrere chirale Atome aufweisen kann oder die hemiacetalische Verknüpfung eine Chiralität aufgrund der asymmetrischen räumlichen Konfiguration der Gesamtheit des Moleküls besitzen kann, Y entweder ein Wasserstoffatom oder eine Gruppe $—CY'_3$, worin Y' ein Chlor- oder Bromatom darstellt, oder eine lineare oder verzweigte Alkylgruppe mit 1 bis 18 Kohlenstoffatomen, die gegebenenfalls substituiert ist, darstellt, wobei die βγ-Bindung der Formel I sowie der Substituent Y in diesem letztgenannten Fall einen Teil des Substituenten A bilden kann, sowie der Alkohole der Formel IV

R—OH (IV)

worin R entweder einen Rest eines primären, sekundären oder tertiären Alkohols mit zumindest einem asymmetrischen Kohlenstoffatom oder einen substituierten alkoholischen Rest bedeutet, dessen Chiralität auf die asymmetrische räumliche Konfiguration der Gesamtheit des Moleküls zurückzuführen ist, dadurch gekennzeichnet, daß man Diisobutylaluminiumhydrid in dem Medium eines organischen Lösungsmittels mit einer racemischen oder optisch aktiven Verbindung der allgemeinen Formel II

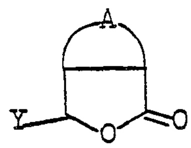

(II)

umsetzt, worin A und Y die vorstehenden Bedeutungen besitzen, danach die entstandene Verbindung der allgemeinen Formel III

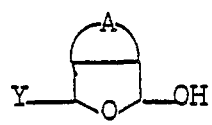

(III)

der Einwirkung eines optisch aktiven oder racemischen Alkohols der allgemeinen Formel IV

R—OH (IV)

worin R die vorstehend angegebene Bedeutung besitzt, unterzieht, um eine Verbindung der Formel I

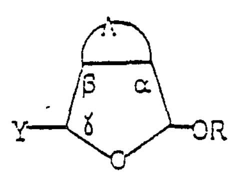

(I)

worin Y, A und R die angegebene Bedeutung besitzen, zu erhalten, deren Diastereomere man trennt, die man spaltet, um die Enantiomeren der hemiacetalischen Verbindung der Formel III zu erhalten, in dem Fall, bei dem man von einer racemischen, hemiacetalischen Verbindung ausging, oder des Alkohols der Formel IV in dem Fall, bei dem man von einem racemischen Alkohol ausging.

2. Verfahren gemäß Anspruch 1, das anwendbar ist, wenn Y ein Wasserstoffatom bedeutet, und das dadurch gekennzeichnet ist, daß man ein Alkaliborhydrid in dem Medium eines Lösungsmittels, danach eine Säure in dem Medium eines organischen Lösungsmittels mit einer Verbindung der allgemeinen Formel V

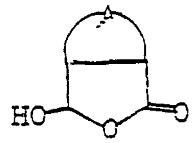

(V)

umsetzt, worin A die vorstehend angegebene Bedeutung besitzt, um die Verbindung der allgemeinen Formel $II_A$

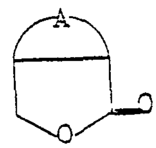

($II_A$)

zu erhalten, die man gemäß den Verfahren des Anspruchs 1 behandelt.

3. Verfahren gemäß Anspruch 1 oder 2, dadurch gekennzeichnet, daß die verschiedenen Atome oder Reste, die das oder die Kohlenstoffatome, die von der Kette A getragen werden oder sich in α- oder β-Stellung befinden, substituieren, ohne Unterschied aus der einen oder anderen der folgenden Gruppen ausgewählt sind :

a) der Gruppe, bestehend aus Wasserstoff, den Halogenatomen, der Nitrogruppe, den Alkylresten mit 1 bis 18 Kohlenstoffatomen, den Cycloalkylresten mit 3 bis 6 Kohlenstoffatomen, dem Phenylrest, den Phenylresten, die entweder durch ein oder mehrere Halogenatome oder durch einen oder mehrere Alkylreste mit 1 bis 6 Kohlenstoffatomen substituiert sind ;

b) der Gruppe, bestehend aus den Resten

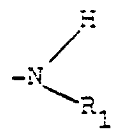

worin die Gruppen $R_1$ ein Wasserstoffatom, einen Alkylrest mit 1 bis 6 Kohlenstoffatomen, den Rest

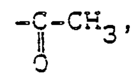

den Rest

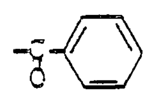

oder den Rest

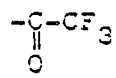

bedeuten ;

c) der Gruppe, bestehend aus den Resten

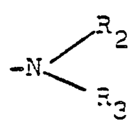

worin $R_2$ und $R_3$, die gleich oder voneinander verschieden sind, einen Alkylrest mit 1 bis 6 Kohlenstoffatomen darstellen oder die Reste $R_2$ und $R_3$ gemeinsam mit dem Stickstoffatom, an das sie gebunden sind, einen Heterocyclus mit 6 Atomen bilden.

4. Verfahren gemäß Anspruch 1, 2 oder 3, dadurch gekennzeichnet, daß die Kette A die folgende Struktur besitzt :

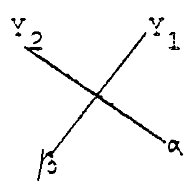

worin $Y_1$ und $Y_2$, die gleich oder voneinander verschieden sind, ein Wasserstoff-, Fluor-, Chlor- oder Bromatom oder einen Alkylrest mit 1 bis 6 Kohlenstoffatomen bedeuten oder $Y_1$ und $Y_2$ gemeinsam mit dem Kohlenstoffatom, an das sie gebunden sind, einen Kohlenstoff-Homocyclus mit 3 bis 7 Kohlenstoffatomen bilden.

5. Verfahren gemäß Anspruch 1, 2, 3 oder 4, dadurch gekennzeichnet, daß die Kette A die folgende Struktur besitzt :

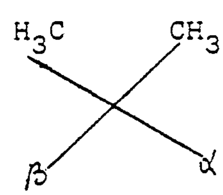

6. Verfahren gemäß Anspruch 1, 2 oder 3, dadurch gekennzeichnet, daß die Kette A eine Struktur, ausgewählt unter der Kette

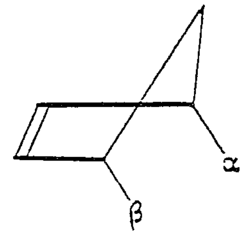

der Kette

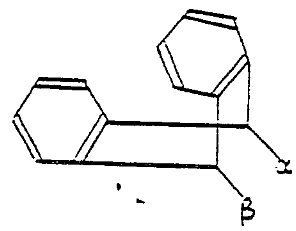

und der Kette

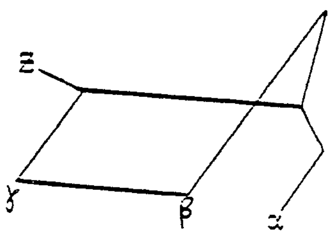

besitzt.

7. Verfahren gemäß einem der Ansprüche 1 bis 6, dadurch gekennzeichnet, daß der Substituent R einen substituierten Cyanomethylrest bedeutet, ausgewählt unter den Resten

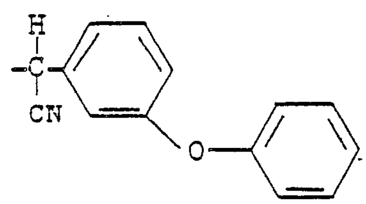

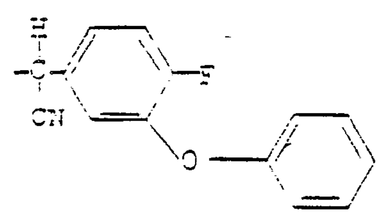

und

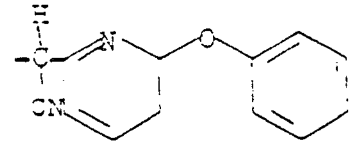

,

sowie den entsprechenden Resten, worin die Cyanogruppe durch einen Alkyl-, Alkenyl- oder Alkinylrest ersetzt ist.

8. Verfahren gemäß einem der Ansprüche 1 bis 6, dadurch gekennzeichnet, daß der Substituent R die folgende Formel besitzt :

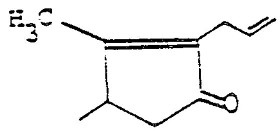

9. Verfahren gemäß einem der Ansprüche 1 bis 7, dadurch gekennzeichnet, daß der Alkohol der Formel IV ausgewählt wird unter

(RS)-α-Cyano-3-phenoxy-benzylalkohol,

(RS)-α-Cyano-4-fluor-3-phenoxy-benzylalkohol,

(RS)-Cyano-6-phenoxy-2-pyridyl-methylalkohol.

10. Verfahren gemäß einem der Ansprüche 1 bis 6 und 8, dadurch gekennzeichnet, daß der Alkohol der Formel IV (RS)-Allethrolon ist.